(19) Europäisches Patentamt
European Patent Office
Office européen des brevets

(11) EP 4 122 496 A1

(12) **EUROPEAN PATENT APPLICATION**
published in accordance with Art. 153(4) EPC

(43) Date of publication:
**25.01.2023 Bulletin 2023/04**

(21) Application number: **21902268.8**

(22) Date of filing: **03.11.2021**

(51) International Patent Classification (IPC):
***A61K 47/42*** (2017.01) ***A61K 9/51*** (2006.01)
***A61P 35/00*** (2006.01) ***B82Y 40/00*** (2011.01)

(52) Cooperative Patent Classification (CPC):
**A61K 9/51; A61K 47/42; A61P 35/00; B82Y 40/00**

(86) International application number:
**PCT/CN2021/128313**

(87) International publication number:
**WO 2022/121561 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.12.2020 CN 202011455391**
**29.09.2021 CN 202111151600**

(71) Applicant: **Suzhou Institute of Nano-tech and Nano-bionics (SINANO) Chinese Academy of Sciences**
**Suzhou, Jiangsu 215123 (CN)**

(72) Inventors:
- **FEI, Hao**
  **Suzhou, Jiangsu 215123 (CN)**
- **JI, Shuangshuang**
  **Suzhou, Jiangsu 215123 (CN)**

(74) Representative: **Cabinet Laurent & Charras**
**Le Contemporain**
**50 Chemin de la Bruyère**
**69574 Dardilly Cedex (FR)**

# (54) POLYPEPTIDE ALBUMIN NANOPARTICLE, PREPARATION METHOD THEREFOR, AND APPLICATION THEREOF

(57) The present application relates to a polypeptide albumin nanoparticle, a preparation method therefor, and an application thereof. The polypeptide albumin nanoparticle is formed by assembling a cationic amphiphilic polypeptide and an albumin. The hydrophobic part of the cationic amphiphilic polypeptide binds to the albumin, and the positive charges carried by the cationic amphiphilic polypeptide can interact with the negative charges on the surface of the albumin, so that the cationic amphiphilic polypeptide and the albumin are assembled to form the polypeptide albumin nanoparticle. In the polypeptide albumin nanoparticle, the cationic amphiphilic polypeptide and the albumin interact with each other, so that stability is improved, hemolytic toxicity is reduced, a high targeting property is provided, tumor cell oncosis can be induced, and the anti-tumor immune response of the body is induced, thereby achieving the effect of providing a high targeting property and efficiently killing tumor cells.

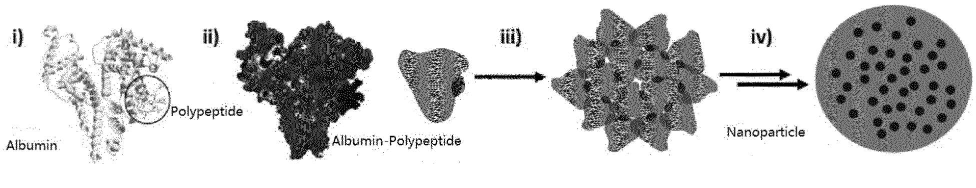


FIG. 1

EP 4 122 496 A1

**Description**

TECHNICAL FIELD

The present application belongs to the technical field of biomedicine and relates to a polypeptide albumin nanoparticle, a preparation method therefor and a use thereof.

BACKGROUND

Cationic amphipathic polypeptides consisting of basic amino acids and hydrophobic amino acids may have electrostatic and hydrophobic interactions with phospholipids and carbohydrate molecules on a biological membrane through their cationic (positive charges) and amphipathic (hydrophilic and hydrophobic) characteristics, so as to exert a cell killing effect by perforation or penetration of the lipid membranes.

A biological membrane is important in function and uniform in structure and irrelevant to genetic mutations. Many studies suggest that drugs based on a membrane perforation or penetration killing mechanism cause no drug resistance of tumor, and cationic amphipathic polypeptides containing more cations can exert a higher selective killing effect on tumor cells that have more negative charges on their surfaces than normal cells. Therefore, cationic amphipathic polypeptides are believed to have a broad development prospect in the field of anti-tumor drugs. However, the cationic amphipathic polypeptides easily cause hemolysis reactions and have problems in aspects of *in vivo* kinetics, targeting and toxic side effects, which limit their application to the preparation of anti-tumor drugs.

The current medicinal optimization methods of cationic amphipathic polypeptides mainly include chemical modifications and the use of a carrier system. The chemical modifications, such as the replacement of L-type amino acids with non-natural D-type amino acids, polypeptide cyclization and the preparation of peptidomimetics with side chains retained and main peptide chains modified, can resist enzymatic degradation to some extent but cannot solve the problems of safety and tumor targeting, and the economic cost of D-type polypeptides could be high. PEGylation has the advantages of reducing non-specific uptake in tissues and cytotoxicity, increasing blood half-life by reducing peptide degradation, but often also reduces the activity of these peptides. Lipid modification is considered to be the most advantageous method for medicalizing cationic amphipathic polypeptides. A subtle change of a lipidation strategy, including fatty acid type, lipid anchor position or interval insertion, has a profound effect on the physicochemical properties, biological activity and pharmacokinetics of lipidated peptides. However, the lipidation strategy is designed by a complex process and with high difficulty.

Another medicalization strategy is to use carrier systems to improve the stability, toxicity, half-life and targeting of cationic amphipathic polypeptides. However, due to structural similarity, some polymer micelles or liposomes that are also composed of monomers containing hydrophobic and hydrophilic ends may be damaged by cationic amphipathic polypeptides, which increases delivery difficulty to some extent. At present, such carrier systems are still in the stage of basic studies. Researchers have reported the use of silica nanoparticles as a drug loading system loaded with antimicrobial peptides LL-37. Although the antimicrobial peptides LL-37 are loaded into the silica nanoparticles to improve the resistance of polypeptides to enzymatic degradation, there are still some problems such as low drug loading capacity and hemolytic toxicity (Braun K, Pochert A, Lindén Mika, et al. Membrane interactions of mesoporous silica nanoparticles as carriers of antimicrobial peptides [J]. Journal of Colloid & Interface, 2016, 475: 161-170). Huang et al. have reported that an $\alpha$-helical peptide capable of modulating a nanostructure is ligated to theN-terminal of a cationic amphipathic polypeptide melittin so that cations of melittin are deeply embedded in phospholipids of a liposome, and the hemolytic effect of melittin can be shielded to some extent, while this method of ligating another polypeptide changes the physicochemical properties of the polypeptide and the range of adaptation needs to be further verified (Huang C, Jin H, Qian Y, et al. Hybrid melittin cytolytic Peptide-driven ultrasmall lipid nanoparticles block melanoma growth in vivo. [J]. ACS Nano, 2013, 7(7): 5791-800). It has also been reported in studies that a membrane-splitting peptide cTL is ligated by an Au-S bond to a gold nanocage whose outer layer is modified with SH-mPEG. Such nanoparticle has significantly improved hemolytic toxicity after being irradiated with near infrared light and can damage both irradiated and un-irradiated tumor cells. However, such polypeptide-loading metal system have a potential risk of cytotoxicity to non-tumor cells when triggered by light/near infrared light and its safety needs to be further evaluated (Ji-Gang Piao, Dong Liu, Kan Hu, et al. Cooperative Nanoparticle System for Photothermal Tumor Treatment without Skin Damage [J]. ACS Applied Materials & Interfaces, 2016, 8(4): acsami.5b11664).

In summary, the problems of unstable drug loading system, high preparation difficulty and uncertain biological safety of the drug carrier systems exist in the existing art. It is therefore significant for the preparation of anti-tumor drugs to provide cationic amphipathic polypeptides with good biological safety, stability, tumor targeting and tumor killing effect, which can be applied in cationic membrane-active anti-tumor drug preparation and help achieve *in vivo* drug delivery and therapeutic application.

SUMMARY

The present application provides a polypeptide albumin nanoparticle, a preparation method therefor and a use thereof. The polypeptide albumin nanoparticle has relatively high stability, tumor targeting capacity and a low hemolytic toxic side effect, kills tumor cells through an oncosis mechanism and induces an anti-tumor immune response of the host, and has a broad development prospect in the preparation of an anti-tumor drug.

In a first aspect, the present application provides a polypeptide albumin nanoparticle. The polypeptide albumin nanoparticle is assembled from a cationic amphipathic polypeptide and serum albumin.

In the present application, a hydrophobic part of the cationic amphipathic polypeptide binds to the albumin, and positive charges carried by the cationic amphipathic polypeptide can interact with negative charges on the surface of the albumin so that the cationic amphipathic polypeptide and the albumin are assembled into the polypeptide albumin nanoparticle (the assembly schematic diagram is shown in FIG. 1). The polypeptide albumin nanoparticle is formed through assembly, and the cationic amphipathic polypeptide is embedded in the albumin so that the cationic amphipathic polypeptide is prevented from being in contact with degradation enzymes and erythrocyte membranes, improving the stability and safety of the cationic amphipathic polypeptide.

In the present application, the polypeptide albumin nanoparticle is nano-scaled and has a high permeability and long retention effect, the albumin therein is actively absorbed and enriched by tumor cells so that the polypeptide albumin nanoparticle has a high targeting property, and at the same time, the polypeptide albumin nanoparticle can induce the oncosis of tumor cells and induce the anti-tumor immune response of the host, thereby achieving the high targeting property and efficiently killing the tumor cells.

Preferably, the cationic amphipathic polypeptide includes a hydrophobic part and a hydrophilic part.

Preferably, the hydrophilic part includes any one or a combination of at least two of arginine (Arg, R), lysine (Lys, K) or histidine (His, H), in which a typical but non-limiting combination includes a combination of arginine and lysine, a combination of arginine and histidine or a combination of arginine, lysine and histidine, preferably arginine.

Preferably, the hydrophobic part includes any one or a combination of at least two of an $[Ir(ppy)_2(H_2O)_2]OTf$ complex, a hydrophobic amino acid domain or a lipid domain, in which a typical but non-limiting combination includes a combination of $[Ir(ppy)_2(H_2O)_2]OTf$ and the hydrophobic amino acid or a combination of the hydrophobic amino acid and the lipid domain.

Preferably, the hydrophobic amino acid domain includes any one or a combination of at least two of phenylalanine (Phe, F), leucine (Leu, F), isoleucine (Ile, I), tryptophan (Trp, W), valine (Val, V), methionine (Met, M) or alanine (Ala, A), in which a typical but non-limiting combination includes a combination of phenylalanine, leucine, isoleucine and tryptophan, a combination of phenylalanine, leucine and tryptophan or a combination of leucine, isoleucine, tryptophan and valine.

Preferably, the lipid domain includes any one or a combination of at least two of cholesterol and a derivative thereof or a fatty acid and a derivative thereof.

Preferably, the cationic amphipathic polypeptide has a structural formula represented by Formula I or Formula II, wherein n is the number of arginine residues and an integer from 1 to 9, which includes, but is not limited to, 2, 3, 5, 6, 7 or 8, preferably from 5 to 9, further preferably 8.

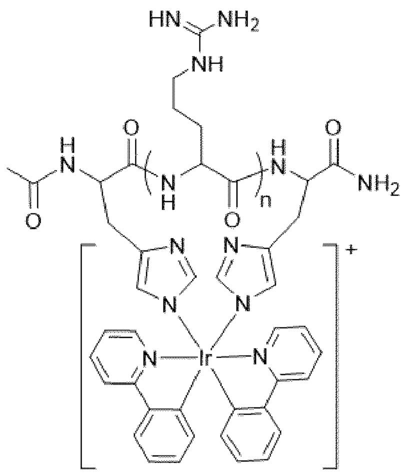

Formula I

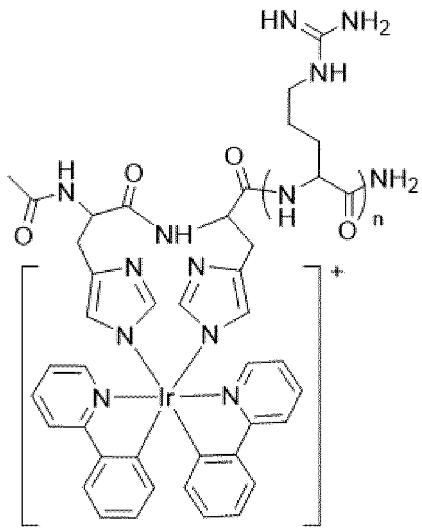

Formula II.

In the present application, a hydrophobic iridium complex ligand $[Ir(ppy)_2(H_2O)_2]OTf$ reacts to hydrophilic oligoarginine to obtain an amphipathic iridium-coordinated oligoarginine polypeptide. The amphipathic iridium-coordinated oligoarginine polypeptide can interact with phospholipids and carbohydrate molecules on a biological membrane through electrostatic and hydrophobic interactions, followed by exerting a cytotoxic effect via perforation or penetration of the cell membranes, thereby enhancing an ability of inhibiting the activity of tumor cells.

Preferably, the cationic amphipathic polypeptide has a primary structure of $CH_3CO-XR_n-CONH_2$ or lipid-$R_n-CONH_2$, wherein X includes any one or a combination of at least two of Phe, Leu, Ile, Trp, Val, Met or Ala, and n is the number of arginine residues and an integer from 1 to 12, which includes, but is not limited to, 2, 3, 5, 6, 8, 9 or 11, preferably from 3 to 9, further preferably from 5 to 9, and still further preferably 8.

Preferably, X includes 1 to 5 (including, but not limited to, 2, 3 or 4) each of Phe, Leu, Ile, Trp, Val, Met and Ala; preferably 1 to 5 each of Phe, Leu, Ile and Trp.

Preferably, the cationic amphipathic polypeptide has a primary structure of $CH_3CO$-FWLFLRRRRRRRR-$CONH_2$ or chol-RRRRRRRR-$CONH_2$, wherein chol is cholesterol.

Preferably, the albumin includes mammalian serum albumin.

Preferably, the mammalian serum albumin includes human serum albumin (HSA) and/or bovine serum albumin.

In a second aspect, the present application provides a preparation method for the polypeptide albumin nanoparticle in the first aspect. The preparation method includes:

Preparing a cationic amphipathic polypeptide solution and an albumin solution, separately and mixing the cationic amphipathic polypeptide solution and the albumin solution at a molar ratio to obtain the polypeptide albumin nanoparticle.

Preferably, the cationic amphipathic polypeptide solution has a concentration of 20-5000 μM, which includes, but is not limited to, 30 μM, 40 μM, 50 μM, 60 μM, 100 μM, 200 μM, 500 μM, 1000 μM, 2000 μM, 3000 μM, 3500 μM, 4000 μM, 4200 μM, 4400 μM, 4800 μM or 4900 μM.

Preferably, the albumin solution has a concentration of 20-5000 μM, which includes, but is not limited to, 30 μM, 40 μM, 50 μM, 60 μM, 100 μM, 200 μM, 500 μM, 1000 μM, 2000 μM, 3000 μM, 3500 μM, 4000 μM, 4200 μM, 4400 μM, 4800 μM or 4900 μM.

Preferably, a molar ratio of cationic amphipathic polypeptides in the cationic amphipathic polypeptide solution to albumin in the albumin solution is (0.1-10):1, which includes, but is not limited to, 0.2:1, 0.8:1, 2:1, 3:1, 4:1, 5:1, 6:1, 7:1, 8:1 or 9:1.

Preferably, the mixing is performed at a temperature of 5-100 °C, which includes, but is not limited to, 6 °C, 7 °C, 9 °C, 10 °C, 15 °C, 30 °C, 40 °C, 50 °C, 70 °C, 80 °C, 85 °C, 90 °C, 92 °C, 94 °C or 98 °C.

Preferably, the mixing is performed for 0.5-120 min, which includes, but is not limited to, 0.6 min, 0.7 min, 0.9 min, 1.5 min, 2 min, 5 min, 10 min, 20 min, 30 min, 50 min, 60 min, 80 min, 90 min, 100 min, 110 min or 115 min.

As a preferred technical solution, the preparation method for the polypeptide albumin nanoparticle includes the following steps:

(1) adding cationic amphipathic polypeptides to water, a trishydroxymethylaminomethane hydrochloride buffer of 40-60 mM or a phosphate buffer of 10-20 mM to obtain a cationic amphipathic polypeptide solution of 20-5000 μM; and adding albumin to water or a phosphate buffer of 10-20 mM to obtain an albumin solution of 20-5000 μM;

(2) mixing the cationic amphipathic polypeptide solution and the albumin solution at a molar ratio of (0.1-10):1 of the cationic amphipathic polypeptides to the albumin, adding water or a phosphate buffer, and mixing the above at 5-100 °C for 0.5-120 min to obtain the polypeptide albumin nanoparticle.

In the present application, the cationic amphipathic polypeptide solution and the albumin solution are mixed under atmospheric pressure so that the polypeptide albumin nanoparticle can be obtained without additional chemical modification, at a low cost and with good repeatability.

In a third aspect, the present application provides a pharmaceutical composition including the polypeptide albumin nanoparticle in the first aspect.

Preferably, the pharmaceutical composition further includes any one or a combination of at least two of a pharmaceutically acceptable carrier, excipient or diluent.

In a fourth aspect, the present application provides a use of the polypeptide albumin nanoparticle in the first aspect or the pharmaceutical composition in the third aspect for preparing an anti-tumor drug.

Compared with the existing art, the present application has the beneficial effects described below.

(1) In the present application, the hydrophobic part of the cationic amphipathic polypeptide binds to the albumin and the positive charges carried by the cationic amphipathic polypeptide can interact with the negative charges on the surface of the albumin so that the cationic amphipathic polypeptide and the albumin are assembled into the polypeptide albumin nanoparticle. In the polypeptide albumin nanoparticle, the cationic amphipathic polypeptide and the albumin collaborate with each other so that the polypeptide albumin nanoparticle has improved stability, reduced hemolytic toxicity and a high targeting property and can induce the oncosis of tumor cells and induce the anti-tumor immune response of the host, thereby achieving the high targeting property and efficient killing of the tumor cells.

(2) The polypeptide albumin nanoparticle of the present application can effectively inhibit enzymatic degradation and has a long elimination half-life in the blood and hence high stability; low hemolysis and high safety; can make tumor cells swell and erupt intracellular contents out and has an oncosis-inducing tumor killing mechanism; can effectively accumulate at tumor sites and has a high targeting property; can promote the maturation of dendritic cells of a lymph node, increase T cells ($CD8^+$) and induce the anti-tumor immune response of the host; and can completely eliminate breast cancer subcutaneous tumor in mice and protect the mice from being re-invaded by tumor cells.

(3) In the present application, the cationic amphipathic polypeptide solution and the albumin solution are mixed under atmospheric pressure so that the polypeptide albumin nanoparticle can be obtained without additional chemical modification, at a low cost and with good repeatability.

BRIEF DESCRIPTION OF DRAWINGS

FIG. 1 is a schematic diagram of the assembly process of polypeptide albumin nanoparticles of the present application, where i) is a polypeptide-albumin binding model, ii) is a surface electrostatic display model of polypeptide albumin complex, and iii) and iv) depict the assembly process of the polypeptide albumin complex into nanoparticles.

FIG. 2 is a graph of hydrated particle size distribution of Ir-cR8-HSA nanoparticles.

FIG. 3 is a graph of hydrated particle size distribution of Ir-cR5-HSA nanoparticles.

FIG. 4 is a graph of hydrated particle size distribution of Ir-aR8-HSA nanoparticles.

FIG. 5 is a graph of hydrated particle size distribution of FR-HSA nanoparticles.

FIG. 6 is a graph of hydrated particle size distribution of CR-HSA nanoparticles.

FIG. 7 is a graph of hydrated particle size distribution of Ir-cr8-HSA nanoparticles.

FIG. 8 is a graph of hydrated particle size distribution of Ir-mK8-HSA nanoparticles.

FIG. 9 is a graph of hydrated particle size distribution of Ir-cK8-HSA nanoparticles.

FIG. 10 is a transmission electron micrograph of Ir-cR8-HSA nanoparticles.

FIG. 11 is a transmission electron micrograph of Ir-cR5-HSA nanoparticles.

FIG. 12 is a transmission electron micrograph of Ir-aR8-HSA nanoparticles.

FIG. 13 is a transmission electron micrograph of FR-HSA nanoparticles.

FIG. 14 is a transmission electron micrograph of CR-HSA nanoparticles.

FIG. 15 is a graph showing the effects of the number of cationic charges of a cationic amphipathic polypeptide and the ratio of cationic amphipathic polypeptides to albumin on the assembly of polypeptide albumin nanoparticles.

FIG. 16 is a graph of hydrated particle size distribution of the assembly of Ir-cR8 and albumin in the presence and absence of a competitive compound.

FIG. 17 is a graph of the inhibitory effects of Ir-cR8 and Ir-cR8-HSA nanoparticles on the activity of 4T1 cells.

FIG. 18 is a graph of the inhibitory effects of Ir-cR5 and Ir-cR5-HSA nanoparticles on the activity of 4T1 cells.

FIG. 19 is a graph of the inhibitory effects of Ir-aR8 and Ir-aR8-HSA nanoparticles on the activity of 4T1 cells.

FIG. 20 is an image showing the oncosis of 4T1 cells incubated with Ir-cR8 and Ir-cR8-HSA nanoparticles.

FIG. 21 is an image showing the oncosis of 4T1 cells incubated with Ir-cR5-HSA nanoparticles.

FIG. 22 is an image showing the oncosis of 4T1 cells incubated with Ir-aR8-HSA nanoparticles.

FIG. 23 is a graph showing the release curves of Ir-cR8 and Ir-cR8-HSA nanoparticles at different pH.

FIG. 24 is a graph showing the serum stability of Ir-cR8 and Ir-cR8-HSA nanoparticles.

FIG. 25 is a graph showing the concentrations of Ir-cR8 and Ir-cR8-HSA nanoparticles in blood at different time points.

FIG. 26 is a graph showing the hemolytic toxicity of Ir-cR8 and Ir-cR8-HSA nanoparticles.

FIG. 27 is a graph showing the distribution of Ir-cR8 and Ir-cR8-HSA nanoparticles in the tissues of mice 24 h after tail vein injection.

FIG. 28 is a graph showing the rates at which PBS, HSA, Ir-cR8 and Ir-cR8-HSA nanoparticles injected through tail veins promote the maturation of dendritic cells in mice.

FIG. 29 is a graph showing the proportions of T cells in tumor tissues.

FIG. 30 is a graph showing the tumor growth curves of experimental groups.

FIG. 31 is a photo of the tumor size of each experimental group and mice in an Ir-cR8-HSA nanoparticle group on the 22th day, where a circle indicates that mice have died before and a box indicates that there is no tumor.

FIG. 32 is a graph showing the tumor growth curves of mice re-inoculated with tumor cells.

FIG. 33 is a photo of mice on the 30th day after re-inoculation with tumor cells.

## DETAILED DESCRIPTION

To further elaborate on the technical means adopted and effects achieved in the present application, the present application is described below in conjunction with examples and drawings. It is to be understood that the specific examples set forth below are intended to explain the present application and not to limit the present application.

Experiments without specific techniques or conditions specified in the examples are conducted according to techniques or conditions described in the literature in the art or a product specification. The reagents or instruments used herein without manufacturers specified are conventional products commercially available from proper channels.

**Example 1**

This example provides a polypeptide albumin nanoparticle. The polypeptide albumin nanoparticle is assembled from an iridium-coordinated oligoarginine polypeptide and human serum albumin (HSA). A preparation method for the polypeptide albumin nanoparticle includes the steps below.

(1) Oligoarginine polypeptide and $[Ir(ppy)_2(H_2O)_2]OTf$ were mixed at a molar ratio of 1:1 at 37 °C for 12 h in a trishydroxymethylaminomethane hydrochloride buffer (Tris-HCl) of 50 mM so that the iridium-coordinated oligoarginine polypeptide (Ir-cR8) was obtained, which is shown by Formula III:

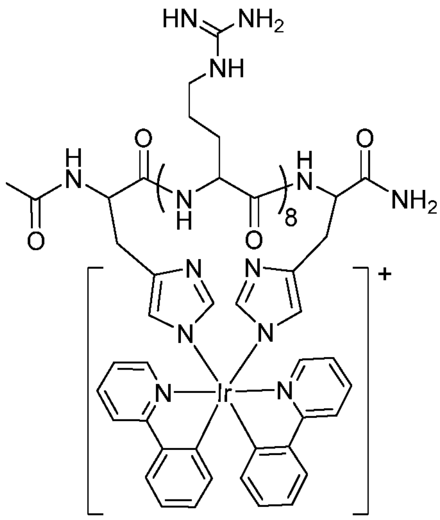

Formula III.

(2) Ir-cR8 was added to ultrapure water to obtain an Ir-cR8 solution of 500 μM, and human serum albumin was dissolved in water and prepared into a human serum albumin solution with the same concentration.

(3) The Ir-cR8 solution and the human serum albumin solution were mixed at a molar ratio of 1:1, an equal volume of phosphate buffer (1×PBS) was added and mixed at room temperature for 2 h, and another equivalent volume of 1×PBS was added to obtain Ir-cR8-HSA nanoparticles.

**Example 2**

This example provides a polypeptide albumin nanoparticle. The polypeptide albumin nanoparticle is assembled from an iridium-coordinated oligoarginine polypeptide and human serum albumin. A preparation method for the polypeptide albumin nanoparticle includes the steps below.

(1) Oligoarginine polypeptides and $[Ir(ppy)_2(H_2O)_2]OTf$ were mixed at a molar ratio of 1:1 at 37 °C for 12 h in a trishydroxymethylaminomethane hydrochloride buffer (Tris-HCl) of 50 mM so that the iridium-coordinated oligoarginine polypeptide (Ir-cR5) was obtained, which is shown by Formula IV:

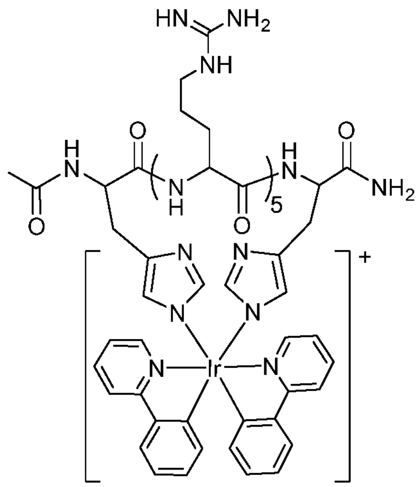

Formula IV.

(2) Ir-cR5 was added to ultrapure water to obtain an Ir-cR5 solution of 500 μM, and human serum albumin was dissolved in water and prepared into a human serum albumin solution with the same concentration.

(3) The Ir-cR5 solution and the human serum albumin solution were mixed at a molar ratio of 3: 1, an equal volume of ultrapure water was added and mixed at room temperature for 2 h, and another equivalent volume of 1×PBS was added to obtain Ir-cR5-HSA nanoparticles.

**Example 3**

This example provides a polypeptide albumin nanoparticle. The polypeptide albumin nanoparticle is assembled from an iridium-coordinated oligoarginine polypeptide and human serum albumin. A preparation method for the polypeptide albumin nanoparticle includes the steps below.

(1) Oligoarginine polypeptide and $[Ir(ppy)_2(H_2O)_2]OTf$ were mixed at a molar ratio of 1:1 at 37 °C for 12 h in a trishydroxymethylaminomethane hydrochloride buffer (Tris-HCl) of 50 mM so that the iridium-coordinated oligoarginine polypeptide (Ir-aR8) was obtained, which is shown by Formula V:

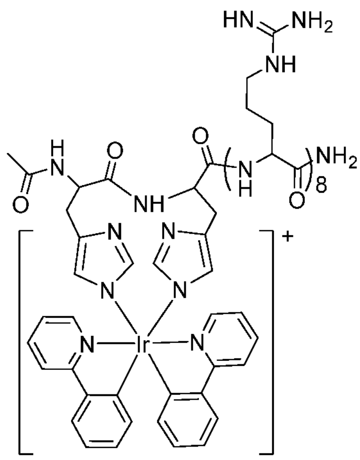

Formula V.

(2) Ir-aR8 was added to ultrapure water to obtain an Ir-aR8 solution of 500 μM, and human serum albumin was dissolved in ultrapure water and prepared into a human serum albumin solution with the same concentration.

(3) The Ir-aR8 solution and the human serum albumin solution were mixed at a molar ratio of 2:1, an equal volume of ultrapure water was added and mixed at room temperature for 2 h, and another equivalent volume of 1×PBS was added to obtain Ir-aR8-HSA nanoparticles.

**Example 4**

This example provides a polypeptide albumin nanoparticle. The polypeptide albumin nanoparticle is assembled from $CH_3CO$-FWLFLRRRRRRRR-$CONH_2$ (FR) and human serum albumin. A preparation method for the polypeptide albumin nanoparticle includes the steps below.

(1) FR was added to ultrapure water to obtain an FR solution of 500 μM, and human serum albumin was dissolved in ultrapure water and prepared into a human serum albumin solution with the same concentration.

(2) The FR solution and the human serum albumin solution were mixed at a molar ratio of 1:1, an equal volume of ultrapure water was added and water-bathed at 50 °C for 1 min, 1×PBS was added dropwise until the solution was clear, and then another equivalent volume of ultrapure water was added to obtain FR-HSA nanoparticles.

**Example 5**

This example provides a polypeptide albumin nanoparticle. The polypeptide albumin nanoparticle is assembled from chol-RRRRRRRR-$CONH_2$ (CR) and human serum albumin. A preparation method for the polypeptide albumin nanoparticle includes the steps below.

(1) CR was added to ultrapure water to obtain a CR solution of 500 μM, and human serum albumin was dissolved in ultrapure water and prepared into a human serum albumin solution with the same concentration.

(2) The CR solution and the human serum albumin solution were mixed at a molar ratio of 2:1, an equal volume of ultrapure water was added and water-bathed at 50 °C for 1 min, 1×PBS was added dropwise until the solution was clear, and then another equivalent volume of ultrapure water was added to obtain CR-HSA nanoparticles.

**Example 6**

This example provides a polypeptide albumin nanoparticle. The polypeptide albumin nanoparticle is assembled from an iridium-coordinated oligoarginine polypeptide and human serum albumin. A preparation method for the polypeptide albumin nanoparticle includes the steps below.

(1) Oligoarginine (D-type) polypeptide $CH_3CO$-hrrrrrrrrh-$CONH_2$ and $[Ir(ppy)_2(H_2O)_2]OTf$ were mixed at a molar ratio of 1:1 at 37 °C for 2 h in a trishydroxymethylaminomethane hydrochloride buffer (Tris-HCl) of 50 mM so that the iridium-coordinated oligoarginine polypeptide (Ir-cr8) was obtained, which is shown by Formula VI:

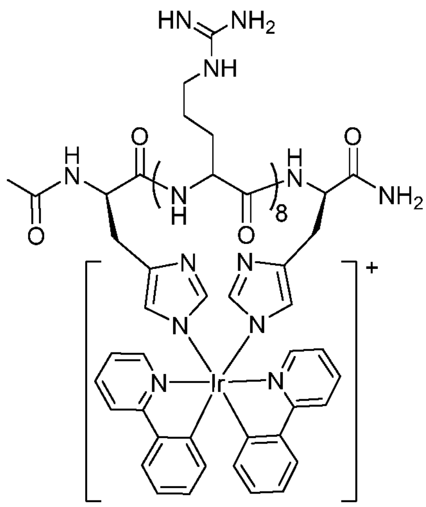

Formula VI.

(2) Ir-cr8 was added to ultrapure water to obtain an Ir-cr8 solution of 500 μM, and human serum albumin was dissolved in water and prepared into a human serum albumin solution with the same concentration.

(3) The Ir-cr8 solution and the human serum albumin solution were mixed at a molar ratio of 1:1, an equal volume of ultrapure water was added and mixed at room temperature for 2 h, and another equivalent volume of 1×PBS was added to obtain Ir-cr8-HSA nanoparticles.

**Example** 7

This example provides a polypeptide albumin nanoparticle. The polypeptide albumin nanoparticle is assembled from an iridium-coordinated oligopolypeptide and human serum albumin. A preparation method for the polypeptide albumin nanoparticle includes the steps below.

(1) Oligopolypeptide $CH_3CO$-KKKKHHKKKK-$CONH_2$ and $[Ir(ppy)_2(H_2O)_2]OTf$ were mixed at a molar ratio of 1:1 at 37 °C for 2 h in a trishydroxymethylaminomethane hydrochloride buffer (Tris-HCl) of 50 mM so that the iridium-coordinated oligoarginine polypeptide (Ir-mK8) was obtained, which is shown by Formula VII:

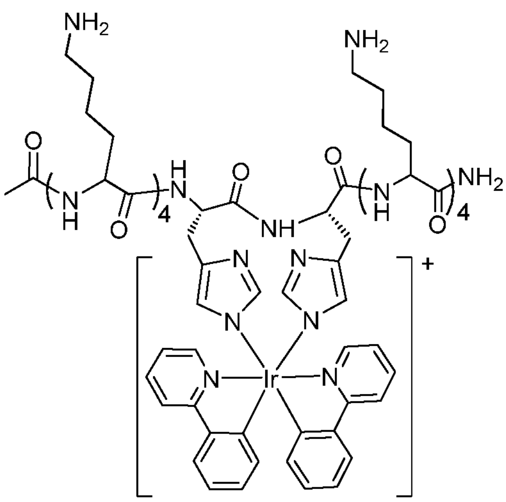

Formula VII.

(2) Ir-mK8 was added to ultrapure water to obtain an Ir-mK8 solution of 500 μM, and human serum albumin was dissolved in water and prepared into a human serum albumin solution with the same concentration.

(3) The Ir-mK8 solution and the human serum albumin solution were mixed at a molar ratio of 1:1, an equal volume of ultrapure water was added and mixed at room temperature for 60 min, and another equivalent volume of 1×PBS was added to obtain Ir-mK8-HSA nanoparticles.

**Example 8**

This example provides a polypeptide albumin nanoparticle. The polypeptide albumin nanoparticle is assembled from an iridium-coordinated oligopolypeptide and human serum albumin. A preparation method for the polypeptide albumin nanoparticle includes the steps below.

(1) Oligopolypeptide $CH_3CO$-HKKKKKKKKH-$CONH_2$ and $[Ir(ppy)_2(H_2O)_2]OTf$ were mixed at a molar ratio of 1:1 at 37 °C for 2 h in a trishydroxymethylaminomethane hydrochloride buffer (Tris-HCl) of 50 mM so that the iridium-coordinated oligoarginine polypeptide (Ir-cK8) was obtained, which is shown by Formula VIII:

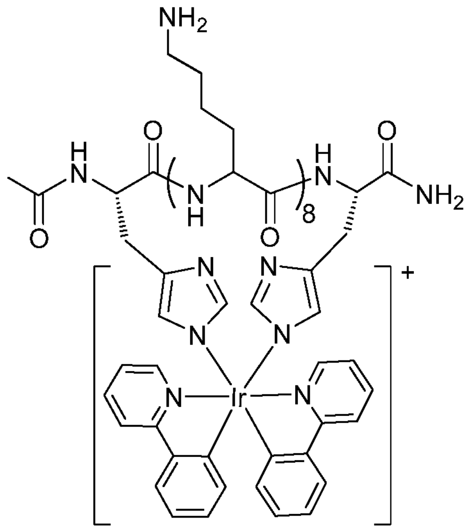

Formula Ⅷ.

(2) Ir-cK8 was added to ultrapure water to obtain an Ir-cK8 solution of 500 μM, and human serum albumin was dissolved in water and prepared into a human serum albumin solution with the same concentration.

(3) The Ir-cK8 solution and the human serum albumin solution were mixed at a molar ratio of 1:1, an equal volume of ultrapure water was added and mixed at room temperature for 60 min, and another equivalent volume of 1×PBS was added to obtain Ir-cK8-HSA nanoparticles.

**Test Example 1**

In this test example, the particle sizes of the polypeptide albumin nanoparticles prepared in Examples 1 to 8 were detected using a dynamic light scattering apparatus and a transmission electron microscope.

Ir-cR8-HSA nanoparticles, Ir-cR5-HSA nanoparticles, Ir-aR8-HSA nanoparticles, Ir-cr8-HSA nanoparticles, Ir-mK8-HSA nanoparticles and Ir-cK8-HSA nanoparticles were diluted to 10 μM with 1×PBS, and FR-HSA nanoparticles and CR-HSA nanoparticles were diluted to 20 μM with 1×PBS. The detection was performed at 25 °C using the dynamic light scattering apparatus, and only the peaks of the formed polypeptide albumin nanoparticles were calculated. The results are shown in FIGS. 2 to 9.

As can be known from FIGS. 2 to 9, the average hydrated particle size of the Ir-cR8-HSA nanoparticle is 155.6 nm, the average hydrated particle size of the Ir-cR5-HSA nanoparticle is 240.6 nm, the average hydrated particle size of the Ir-aR8-HSA nanoparticle is 258.7 nm, the average hydrated particle size of the Ir-cr8-HSA nanoparticle is 144.9 nm, the average hydrated particle size of the Ir-mK8-HSA nanoparticle is 266.5 nm, the average hydrated particle size

of the Ir-cK8-HSA nanoparticle is 322.4 nm, the average hydrated particle size of the FR-HSA nanoparticle is 396.1 nm, and the average hydrated particle size of the CR-HSA nanoparticle is 295.3 nm.

Ir-cR8-HSA nanoparticles, Ir-cR5-HSA nanoparticles, Ir-aR8-HSA nanoparticles, FR-HSA nanoparticles and CR-HSA nanoparticles were diluted to 500 μM with 1×PBS and dropped on the carbon-supported grid with 300 meshes, separately. When the liquid droplets were about to be dried, one drop of 3% phosphotungstic acid dye solution was added, negative staining was performed for 1-2 min, the dye solution was absorbed, and the nanoparticles were washed with water three times, dried in the air, and analyzed using the transmission electron microscope. The results are shown in FIGS. 10 to 14.

As can be known from FIGS. 10 to 14, the particle sizes of the Ir-cR8-HSA nanoparticles are distributed within 40-100 nm, the particle sizes of the Ir-cR5-HSA nanoparticles are distributed within 50-200 nm, the particle sizes of the Ir-aR8-HSA nanoparticles are distributed within 40-200 nm, the particle sizes of the FR-HSA nanoparticles are distributed within 40-400 nm, and the particle sizes of the CR-HSA nanoparticles are distributed within 40-400 nm.

**Test Example 2**

In this test example, the effects of the number of charges of a cationic amphipathic polypeptide and the ratio of cationic amphipathic polypeptides to human serum albumin on the assembly of polypeptide albumin nanoparticles were investigated.

Each of Ir-cR3 (as shown by Formula IX), Ir-cR5 and Ir-cR8 was mixed with human serum albumin at a molar ratio of 2:1, 1:1, 1:2 and 1:3, separately. The final concentration of the human serum albumin was 10 μM. The hydrated particle size distribution of the mixed solution of polypeptides and albumin was detected using a dynamic light scattering apparatus. The results are shown in FIG. 15. As can be seen, the large the number of positive charges (arginine) in the hydrophilic part of the cationic amphipathic polypeptide and the smaller the ratio of the albumin, the easier the cationic amphipathic polypeptides and albumin are assembled into the polypeptide albumin nanoparticles.

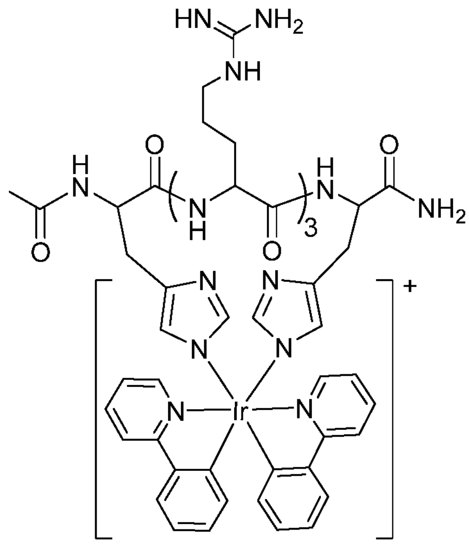

Formula IX

**Test Example 3**

In this test example, the inhibitory ability of iridium-coordinated oligoarginine polypeptides on the activity of tumor cells was detected.

Human cervical cancer cells, Hela cells, were dispersed with a DMEM medium containing 10% fetal bovine serum and spread to a 96-well plate, 8000 cells per well. After the cells were attached to the walls, Ir-cR5, Ir-cR8 and Ir-aR8 of 2, 4, 8, 16, 32, 64 and 128 μM and the corresponding oligoarginine polypeptides cR5, cR8 and aR8 were added and incubated for 24 h. The cell activity was detected by a thiazole blue method and the half-maximal inhibitory concentration ($IC_{50}$) was calculated. The results are shown in Table 1: compared with the oligoarginine polypeptides that are not coordinated, the iridium-coordinated oligoarginine polypeptides have a significantly reduced half-maximal inhibitory concentration ($IC_{50}$) and significantly increased killing toxicity for Hela cells.

Table 1

| Experimental Group | $IC_{50}$ (μM) |
|---|---|
| Ir-cR5 | 23.5 |
| Ir-cR8 | 7.3 |
| Ir-aR8 | 7.3 |
| cR5 | >100 |
| cR8 | >100 |
| aR8 | >100 |

**Test Example 4**

Human serum albumin is a single chain polypeptide and can be divided into two subdomains, A and B, and each subdomain can be further divided into three regions: I, II and III. The human serum albumin has three major drug binding sites, which are drug binding site 1 (also referred to as Sudlow site 1) at IIA, drug binding site 2 (also referred to as Sudlow site 2) at IIIA, and drug binding site 3 at I B, respectively. In this test example, the binding position of Ir-cR8 on the human serum albumin was detected. An aqueous solution (10 μM) of human serum albumin and the mixed solutions (10 μM) of human serum albumin and competitive compounds of the drug binding sites 1, 2 and 3 of the human serum albumin, phenylbutazone, ibuprofen and lidocaine, were prepared. After being mixed for 5 min, the solutions were added with 0, 2, 4, 6, 8 and 10 μM Ir-cR8, separately and excited with light at 280 nm. The excitation spectra of the solutions at 300-450 nm were scanned and the binding constant and the number of binding sites of Ir-cR8 to albumin in the presence and absence of the competitive compounds were calculated according to Formula (1):

$$lg\,(F0 - F)/F = lgKa + nlgc \qquad (1);$$

wherein, F0 denotes the fluorescence intensity of the system in the absence of the competitive compound, F denotes the fluorescence intensity of the system in the presence of the competitive compound, Ka denotes the binding constant, c denotes the concentration of a quencher, and n denotes the number of binding sites.

The results are shown in Table 2. As can be seen, the number of binding sites of Ir-cR8 on the human serum albumin is 1 and Ir-cR8 mainly binds at site 1.

Table 2

| Competition Compound | Binding Constant Kb (L/mol) | Number of Binding Sites (n) |
|---|---|---|
| \ | $1.33 \times 10^6$ | 1.19 |
| Phenylbutazone | $1.09 \times 10^6$ | 1.18 |
| Ibuprofen | $1.69 \times 10^6$ | 1.22 |
| Lidocaine | $1.35 \times 10^6$ | 1.20 |

To further verify the above conclusion, 10 μM human serum albumin was mixed with the competitive compound at each site at a molar ratio of 1:4, and then 10 μM Ir-cR8 was added. The mixed solution of human serum albumin without the competitive compound and Ir-cR8 with the same concentration was used as control, and the assembly of polypeptide albumin nanoparticles in the presence and absence of the competitive compound was detected using a dynamic light scattering apparatus. The results are shown in FIG. 16 and further verify that the main binding site of Ir-cR8 on the human serum albumin is site 1.

**Test Example 5**

The tumor killing ability and mechanism of Ir-cR8-HSA nanoparticles, Ir-cR5-HSA nanoparticles and Ir-aR8-HSA nanoparticles were studied.

The breast cancer cells 4T1 of mice were dispersed with a DMEM medium containing 10% fetal bovine serum

and spread to a 96-well plate, 8000 cells per well. After the cells were attached to the walls, Ir-cR8-HSA nanoparticles, Ir-cR5-HSA nanoparticles and Ir-aR8-HSA nanoparticles and Ir-cR8, Ir-cR5 and Ir-aR8 of 2, 4, 8, 16, 32, 64 and 128 μM were added separately and incubated for 24 h. The cell activity was detected by a thiazole blue method. The results are shown in FIGS. 17 to 19. As can be seen, the half-maximal inhibitory concentrations of Ir-cR8 and Ir-cR8-HSA nanoparticles are 10.1 μM and 13.9 μM, respectively, the half-maximal inhibitory concentrations of Ir-cR5 and Ir-cR5-HSA nanoparticles are 25.45 μM and 29.64 μM, respectively, and the half-maximal inhibitory concentrations of Ir-aR8 and Ir-aR8-HSA nanoparticles are 13.16 μM and 16.20 μM, respectively, indicating that the preparation of cationic amphipathic polypeptides into polypeptide albumin nanoparticles has no significant effect on the killing activity for tumor cells.

A 15 mm glass bottom cell culture dish was added with $3\times10^5$ 4T1 cells. After the cells were attached to the walls, 1 mL half-maximal inhibitory concentrations of Ir-cR8-HSA nanoparticles, Ir-cR5-HSA nanoparticles, Ir-aR8-HSA nanoparticles and Ir-cR8 (each containing 5 μg/mL propidium iodide) was added separately, and laser confocal scanning microscopy was performed at 405 nm and 561 nm to observe the cell death. The results are shown in FIGS. 20 to 22: tumor cells swell and erupt intracellular contents out, indicating that the polypeptide albumin nanoparticles have an oncosis-inducing tumor killing mechanism.

**Test Example 6**

In this test example, the stability and safety of Ir-cR8-HSA nanoparticles were analyzed.

500 μL of Ir-cR8 and Ir-cR8-HSA nanoparticles (200 μM) were put in a dialysis bag with a molecular weight of 8000-14000 D, separately. The dialysis bag was placed in a 15 mL centrifuge tube. Each centrifuge tube was added with 5 mL of PBS buffer with a pH of 5.0, 6.5 and 7.4 and placed in a 37 °C and 200 rpm thermostatic oscillator. 500 μL of buffer was taken out and 500 μL of fresh buffer was supplemented to the centrifuge tube after 1 h, 2 h, 4 h, 6 h, 16 h and 24 h. The taken buffer was excited at a wavelength of 328 nm so that the emission spectrum of the buffer at 350-550 nm was detected. The concentration of Ir-cR8 in the buffer was quantified according to the fluorescence standard curve of Ir-cR8 in the buffer. The results are shown in FIG. 23. It can be seen that Ir-cR8 in the Ir-cR8-HSA nanoparticles is more difficult to release than free Ir-cR8.

100 μL of Ir-cR8 and Ir-cR8-HSA nanoparticles (160 μM) were mixed with 900 μL of PBS solution containing 10% fetal bovine serum and incubated for 2 h, 4 h, 16 h and 24 h, separately. The incubated mixed solution was added to a 96-well plate with 4T1 cells spread and incubated for 24 h, and thiazole blue was added to detect cell inhibitory activity. The results are shown in FIG. 24. Compared with Ir-cR8 and Ir-cR8-HSA nanoparticles that are not treated with serum, the Ir-cR8-HSA nanoparticles retain 87.05% cell inhibitory activity, while Ir-cR8 only remain 42.74% cell inhibitory activity, indicating that the preparation of polypeptide albumin nanoparticles can inhibit the degradation of cationic amphipathic polypeptides by various enzymes in serum and facilitate the *in vivo* circulation of cationic amphipathic polypeptides. 36 BALB/c mice were equally divided into two groups and injected with 100 μL of Ir-cR8 and Ir-cR8-HSA nanoparticles (500 μM) through tail veins, separately. After 0.5 h, 2 h, 4 h, 8 h, 24 h and 48 h, eyes were removed for blood sampling and centrifuged at 10000 rpm for 10 min. 100 μL of centrifuged serum was added with 1 mL of high chlorine acid and 3 mL of aqua regia, digested at 260 °C for 2h, and added with water to 10 mL. The content of metal iridium in the blood at each time point was detected by ICP-MASS. The results are shown in FIG. 25. The results show that the elimination half-life of Ir-cR8 in the blood is less than 0.5 h, while the elimination half-life of Ir-cR8-HSA nanoparticles in the blood is greater than 15 h so that the circulating time of Ir-cR8-HSA nanoparticles in the blood is significantly prolonged.

1 mL of mouse blood was added with 1 mL PBS, dispersed well and centrifuged at 1000 rpm for 5 min. The above steps were repeated. PBS was discarded, 52.5 mL of fresh PBS was added, a blank control group (140 μL of PBS/well), a negative control group (70 μL of erythrocyte suspension+70 μL of PBS/well), a positive control group (70 μL of pure water lysate of erythrocyte+70 μL of pure water/well) and an experimental group (70 μL of Ir-cR8 or Ir-cR8-HSA nanoparticles+70 μL of erythrocyte suspension) were set, shaken at 37 °C and 87 rpm for 2 h, and centrifuged at 3000 rpm for 5 min. 90 μL of supernatant was detected for an absorbance value at 405 nm. The results are shown in FIG. 26: Ir-cR8 with a concentration of 78.125 μM causes 52.87% hemolysis, while Ir-cR8-HSA nanoparticles with a concentration of 625 μM cause only 15.70% hemolysis, indicating that the preparation of cationic amphipathic polypeptides into polypeptide albumin nanoparticles can significantly reduce the hemolysis effect of the polypeptides themselves and improve biological safety.

**Test Example 7**

In this test example, the tumor targeting property of Ir-cR8-HSA nanoparticles and their effect of stimulating the anti-tumor immune response of the host were studied.

The groins of BALB/c mice were subcutaneously injected with $10^6$ 4T1 cells to construct a BALB/c mouse subcutaneous tumor model. After the volume of tumor (volume = $L\times D^2/2$, wherein L denotes the length of tumor and D

denotes the width of tumor) reached 100 $mm^3$, 100 μL of Ir-cR8 and Ir-cR8-HSA nanoparticles (500 μM) were injected into tail veins, separately. 24 h later, the mice were sacrificed, and the heart, liver, spleen, lung, kidney and tumor of the mouse were weighted separately, shredded, added with 1 mL of PBS, homogenized and crushed, and centrifuged at 10000 rpm for 10 min. The supernatant was excited at a wavelength of 328 nm, the fluorescence emission intensity of each sample at 502 nm was measured, and the concentrations of Ir-cR8 and Ir-cR8-HSA nanoparticles in the supernatant of each tissue were calculated according to the fluorescence emission intensities of the PBS solutions of Ir-cR8 and Ir-cR8-HSA nanoparticles at 502 nm under the same excitation conditions. The results are shown in FIG. 27: the concentration of Ir-cR8-HSA nanoparticles at the tumor site is 12.19 μg/g, the concentration of Ir-cR8 at the tumor site is 4.14 μg/g, and 24 h after tail vein injection, the accumulation of Ir-cR8-HSA nanoparticles at the tumor site is about three times that of Ir-cR8, indicating that Ir-cR8-HSA nanoparticles are better accumulated than Ir-cR8 at the tumor site and have a high targeting property.

The groins of BALB/c mice were subcutaneously injected with $10^6$ 4T1 cells to construct a BALB/c mouse subcutaneous tumor model. After the volume of tumor (volume = $L \times D^2/2$) reached 100 $mm^3$, 100 μL of HSA, Ir-cR8 and Ir-cR8-HSA nanoparticles (500 μM) were injected into tail veins for 4 consecutive days, separately, and the same volume of PBS was injected into tail veins as control. On the 5th day after administration, the tumor-draining lymph node near the tumor side was dispersed into single cells, filtered with a 70 μm filter, stained with anti-mouse CD11c-FITC, CD80-PE and CD86-APC antibodies in cell staining buffers for 15 min at 4 °C, washed three times with the cell staining buffer, resuspended with 500 μL of cell staining buffer, and detected by flow cytometry. The results are shown in FIG. 28: Ir-cR8-HSA nanoparticles can accumulate at the tumor and kill tumor cells through oncosis so that dendritic cells (DCs) of the nearby inguinal lymph node absorb tumor-associated antigens and are more mature.

The groins of BALB/c mice were subcutaneously injected with $10^6$ 4T1 cells to construct a BALB/c mouse subcutaneous tumor model. After the volume of tumor (volume = length $\times$ width$^2$/2) reached 100 $mm^3$, 100 μL of HSA, Ir-cR8 and Ir-cR8-HSA nanoparticles (500 μM) were injected into tail veins for 4 consecutive days, separately, and the same volume of PBS was injected into tail veins as control. On the 7th day after administration, the tumor was dispersed into single cells, filtered with a 70 μm filter, stained with anti-mouse CD3-FITC, CD4-Percp-Cy5.5 and CD8a-APC antibodies in cell staining buffers for 15 min at 4 °C, washed three times with the cell staining buffer, resuspended with 500 μL of cell staining buffer, and detected by flow cytometry. The results are shown in FIG. 29: Ir-cR8-HSA nanoparticles promote the maturation of dendritic cells in the inguinal lymph node and the dead tumor cell debris at the tumor site so that cytotoxic T cells ($CD8^+$) at the tumor site significantly increase and an anti-tumor immune response is further enhanced.

$10^6$ 4T1 cells were implanted subcutaneously into the backs of adult female BALB/c mice at the age of six weeks to construct a mouse subcutaneous tumor model. When the volume of tumor reached 50-60 $mm^3$, the mice were divided into 4 groups, five mice in each group, and 100 μL of PBS and HSA, Ir-cR8 and Ir-cR8-HSA nanoparticles (500 μM) were injected into tail veins for 4 consecutive days, separately. The tumor growth of each mouse was observed. The results are shown in FIGS. 30 and 31: the Ir-cR8-HSA nanoparticle group has a good tumor inhibitory effect and can completely eliminate the tumor of the mouse, while other groups cannot effectively inhibit the tumor growth.

Five mice in the Ir-cR8-HSA nanoparticle group whose subcutaneous tumor had been eliminated for the first time were subcutaneously inoculated with $8 \times 10^5$ 4T1 cells on the other side. Five mice at the same age and without any treatment were used as a control group (untreated) and also subcutaneously inoculated with $8 \times 10^5$ 4T1 cells. The tumor growth of the two groups was observed. The results are shown in FIGS. 32 and 33: the mice treated with Ir-cR8-HSA nanoparticles form an anti-tumor immune memory and are not easily subjected to the secondary invasion of tumor, indicating that the polypeptide albumin nanoparticle of the present application can induce the anti-tumor immune response of the host.

To conclude, the polypeptide albumin nanoparticle of the present application can effectively inhibit enzymatic degradation and has a long elimination half-life in blood and high stability; low hemolysis and high safety; can make tumor cells swell and erupt intracellular contents out and has an oncosis-inducing tumor killing mechanism; can effectively accumulate at tumor sites and has a high targeting property; can promote the maturation of dendritic cells of a lymph node, increase T cells ($CD8^+$) and induce the anti-tumor immune response of the host; and can completely eliminate breast cancer subcutaneous tumor in mice and protect the mice from being re-invaded by tumor cells.

The applicant has stated that although the detailed method of the present application is described through the examples described above, the present application is not limited to the detailed method described above, which means that the implementation of the present application does not necessarily depend on the detailed method described above. It should be apparent to those skilled in the art that any improvements made to the present application, equivalent replacements of raw materials of the product of the present application, additions of adjuvant ingredients, selections of specific manners, etc., all fall within the protection scope and the disclosure scope of the present application.

## Claims

1. A polypeptide albumin nanoparticle is assembled from a cationic amphipathic polypeptide and serum albumin.

2. The polypeptide albumin nanoparticle of claim 1, wherein the cationic amphipathic polypeptide comprises a hydrophobic part and a hydrophilic part.

3. The polypeptide albumin nanoparticle of claim 1 or 2, wherein the hydrophilic part comprises any one or a combination of at least two of arginine, lysine or histidine, preferably arginine.

4. The polypeptide albumin nanoparticle of any one of claims 1 to 3, wherein the hydrophobic part comprises any one or a combination of at least two of $[Ir(ppy)_2(H_2O)_2]OTf$, a hydrophobic amino acid or a lipid.

5. The polypeptide albumin nanoparticle of any one of claims 1 to 4, wherein the hydrophobic amino acid comprises any one or a combination of at least two of phenylalanine, leucine, isoleucine, tryptophan, valine, methionine or alanine;

   preferably, the lipid comprises any one or a combination of at least two of cholesterol and a derivative thereof or a fatty acid and a derivative thereof; and
   preferably, the cationic amphipathic polypeptide has a structural formula represented by Formula I or Formula II:

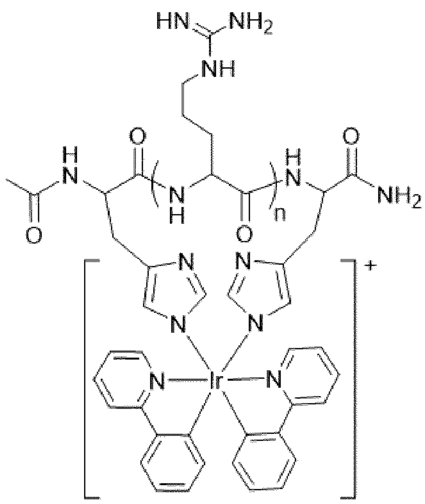

Formula I

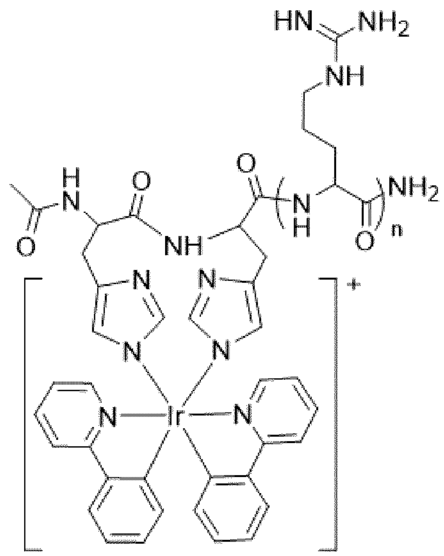

Formula II

wherein n is the number of arginine residues and an integer from 1 to 9;
preferably, the cationic amphipathic polypeptide has a primary structure of $CH_3CO-XR_n-CONH_2$ or lipid-Rn-CONH2;
wherein X comprises any one or a combination of at least two of phenylalanine, leucine, isoleucine, tryptophan, valine, methionine or alanine, n is the number of arginine residues and an integer from 1 to 12.

**6.** The polypeptide albumin nanoparticle of any one of claims 1 to 5, wherein the albumin comprises mammalian albumin; preferably, the mammalian albumin comprises human serum albumin and/or bovine serum albumin.

**7.** A preparation method for the polypeptide albumin nanoparticle of any one of claims 1 to 6, comprising:
preparing a cationic amphipathic polypeptide solution and an albumin solution, separately and mixing the cationic amphipathic polypeptide solution and the albumin solution to obtain the polypeptide albumin nanoparticle.

**8.** The preparation method of claim 7, wherein the cationic amphipathic polypeptide solution has a concentration of 20-5000 μM;
preferably, the albumin solution has a concentration of 20-5000 μM.

**9.** The preparation method of claim 7 or 8, wherein a molar ratio of cationic amphipathic polypeptides to albumin is (0.1-10):1.

**10.** The preparation method of any one of claims 7 to 9, wherein the mixing is performed at a temperature of 5-100 °C; preferably, the mixing is performed for 0.5-120 min.

**11.** The preparation method of any one of claims 7 to 10, comprising the following steps:

(1) adding cationic amphipathic polypeptides to water, a trishydroxymethylaminomethane hydrochloride buffer of 40-60 mM or a phosphate buffer of 10-20 mM to obtain a cationic amphipathic polypeptide solution of 20-5000 μM; and adding albumin to water or a phosphate buffer of 10-20 mM to obtain an albumin solution of 20-5000 μM;
(2) mixing the cationic amphipathic polypeptide solution and the albumin solution at a molar ratio of (0.1-10):1 of the cationic amphipathic polypeptides to the albumin, adding water or a phosphate buffer, and mixing the above at 5-100 °C for 0.5-120 min to obtain the polypeptide albumin nanoparticle.

**12.** A pharmaceutical composition, comprising the polypeptide albumin nanoparticle of any one of claims 1 to 6; preferably, the pharmaceutical composition further comprises any one or a combination of at least two of a pharmaceutically acceptable carrier, excipient or diluent.

**13.** A use of the polypeptide albumin nanoparticle of any one of claims 1 to 6 or the pharmaceutical composition of claim 12 for preparing an anti-tumor drug.

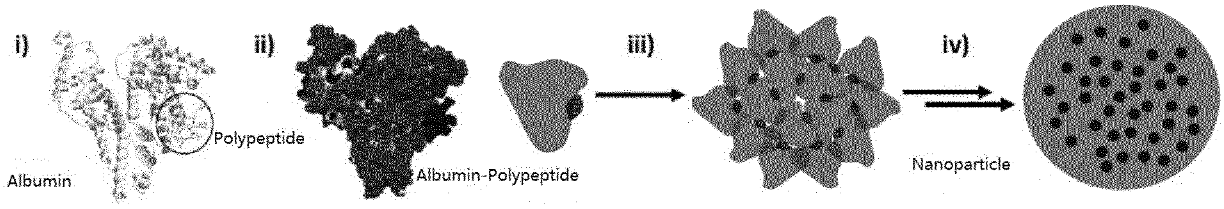


FIG. 1

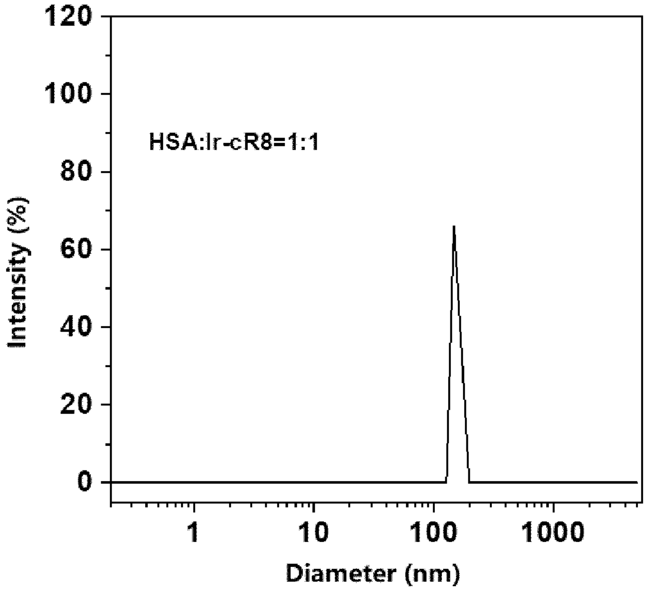


FIG. 2

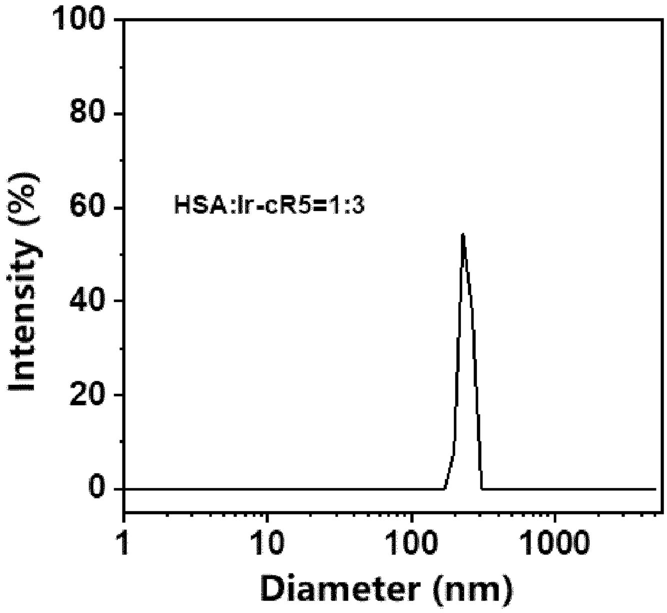


FIG. 3

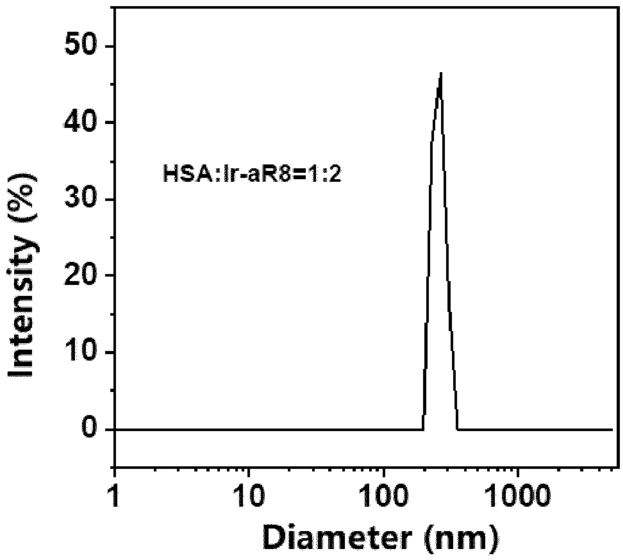
HSA:Ir-aR8=1:2
Intensity (%)
Diameter (nm)
0
10
20
30
40
50
1
10
100
1000

FIG. 4

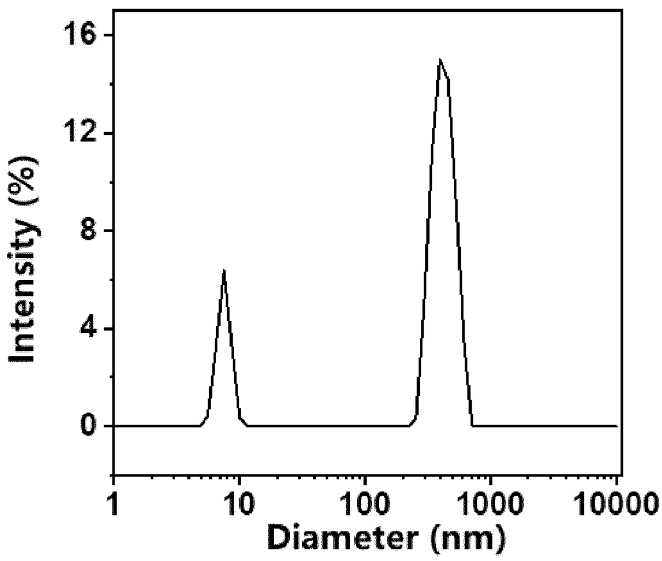
Intensity (%)
Diameter (nm)
0
4
8
12
16
1
10
100
1000
10000

FIG. 5

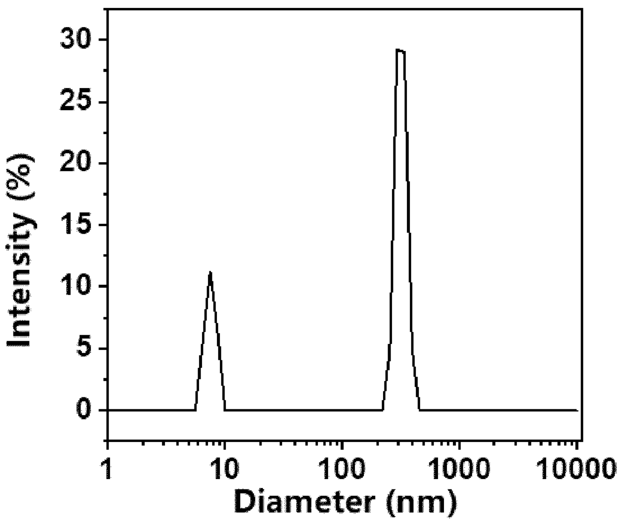
30
25
20
15
10
5
0
Intensity (%)
1
10
100
1000
10000
Diameter (nm)

FIG. 6

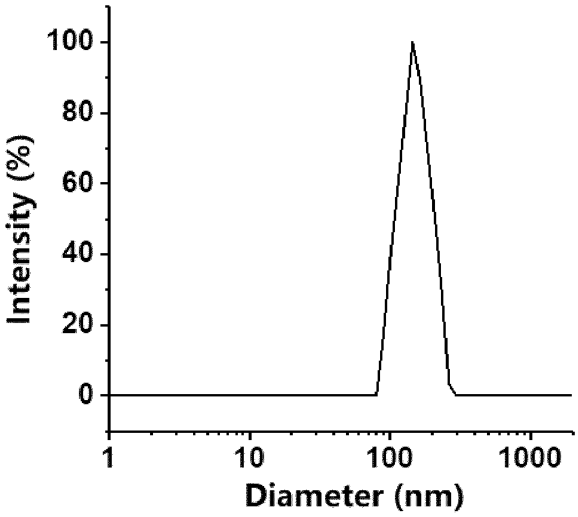
100
80
60
40
20
0
Intensity (%)
1
10
100
1000
Diameter (nm)

FIG. 7

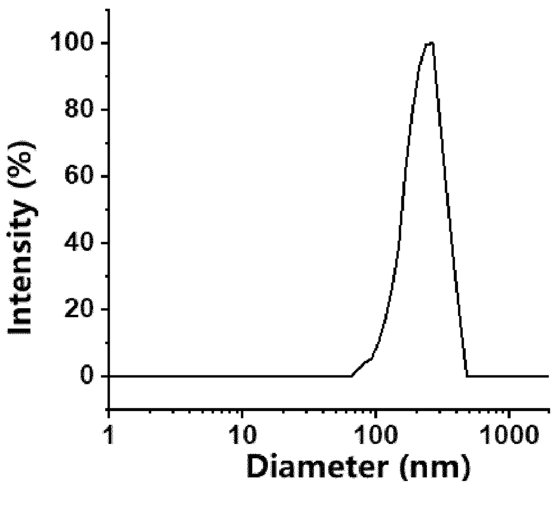
100
80
60
40
20
0
Intensity (%)
1
10
100
1000
Diameter (nm)

FIG. 8

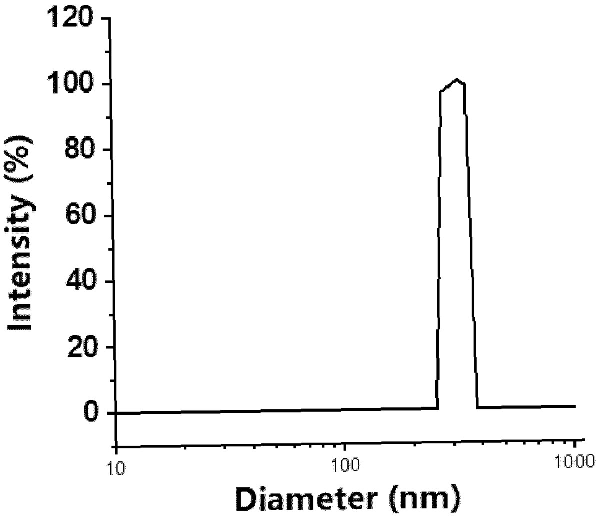


FIG. 9

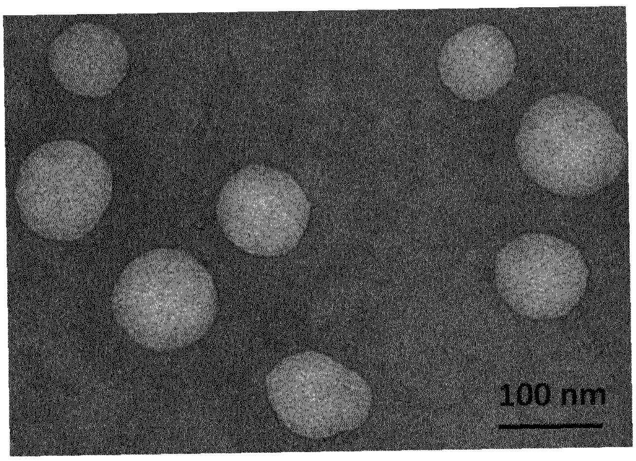


FIG. 10

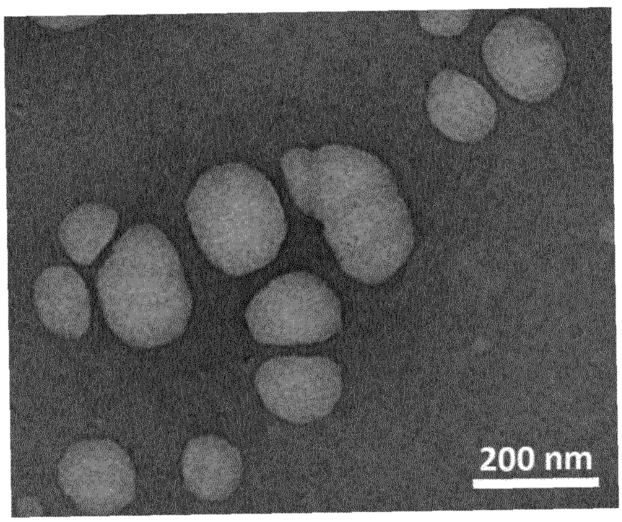


FIG. 11

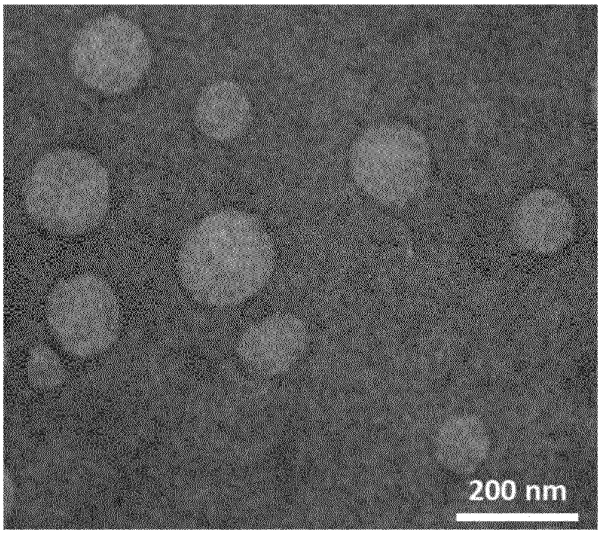


FIG. 12

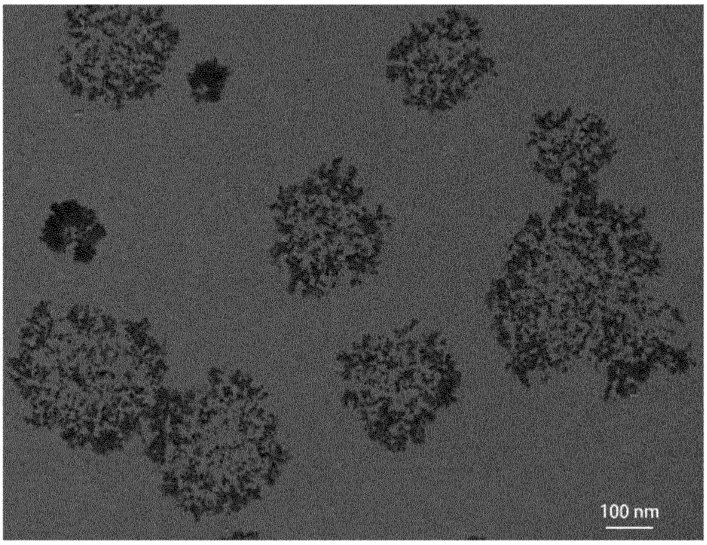


FIG. 13

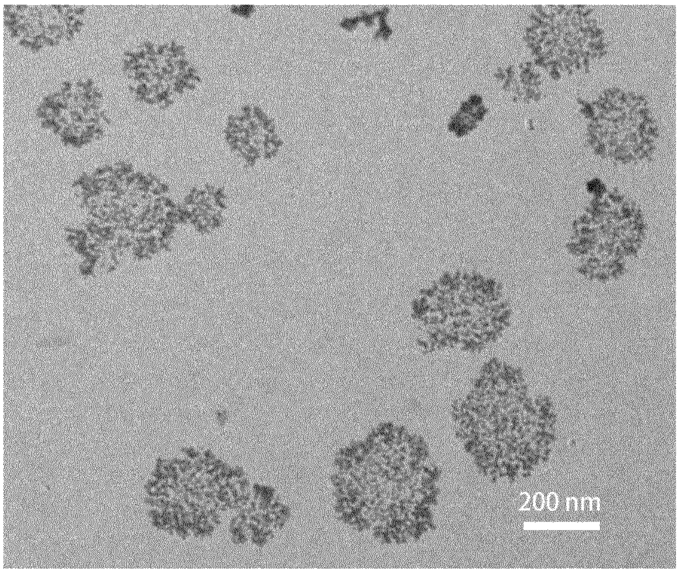

FIG. 14

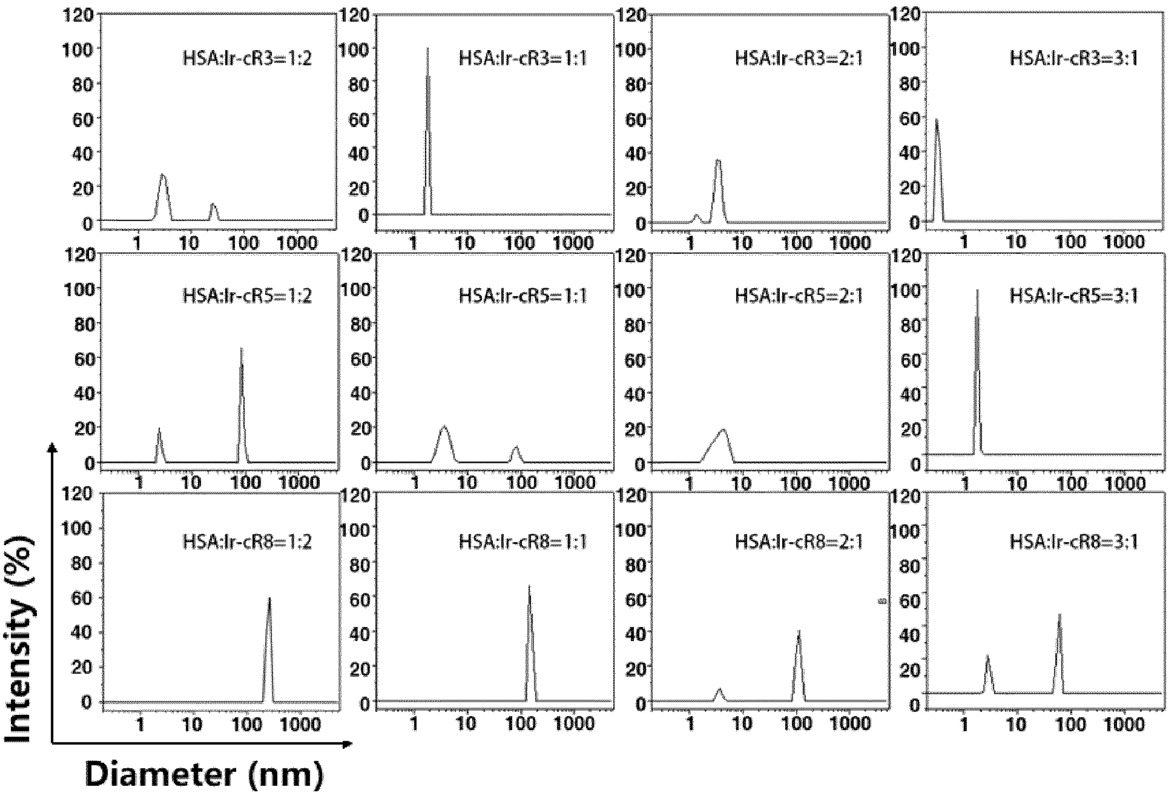


FIG. 15

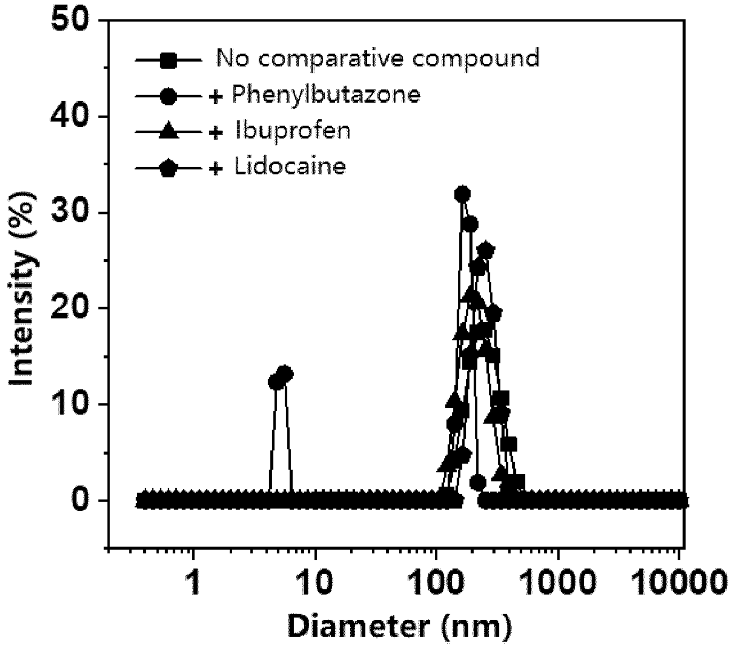


FIG. 16

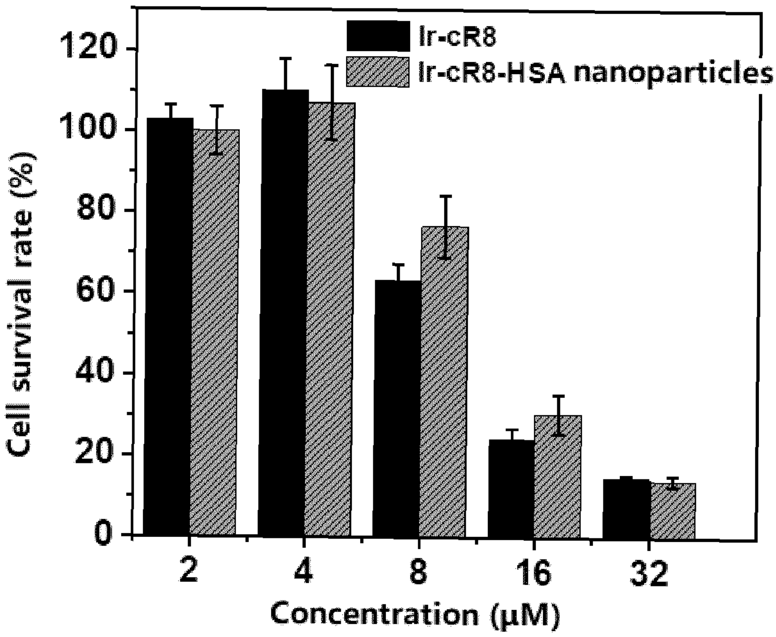
Ir-cR8
Ir-cR8-HSA nanoparticles
120
100
80
60
40
20
0
Cell survival rate (%)
2
4
8
16
32
Concentration (μM)

FIG. 17

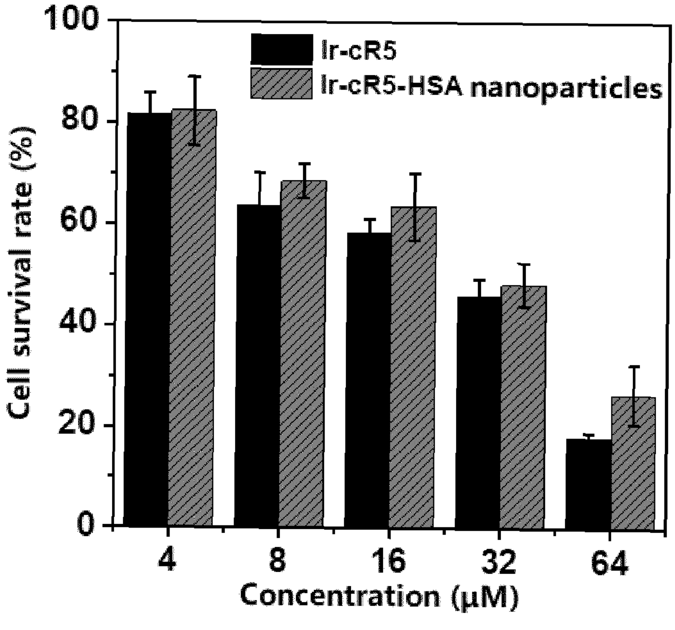
Ir-cR5
Ir-cR5-HSA nanoparticles
100
80
60
40
20
0
Cell survival rate (%)
4
8
16
32
64
Concentration (μM)

FIG. 18

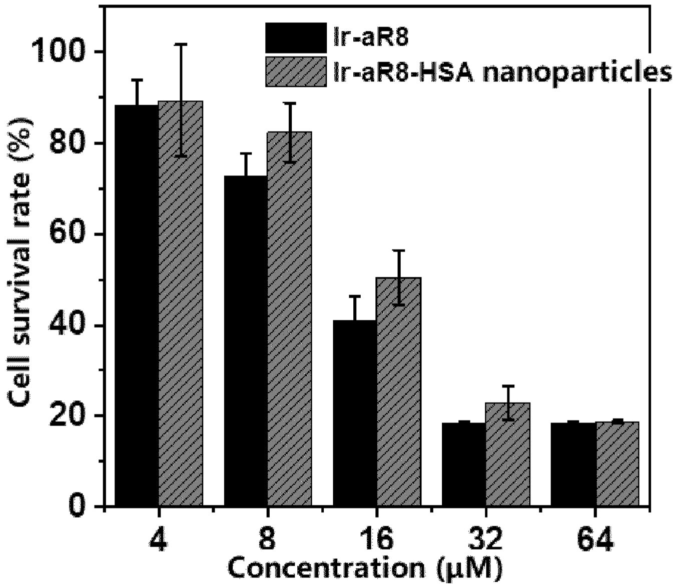


FIG. 19

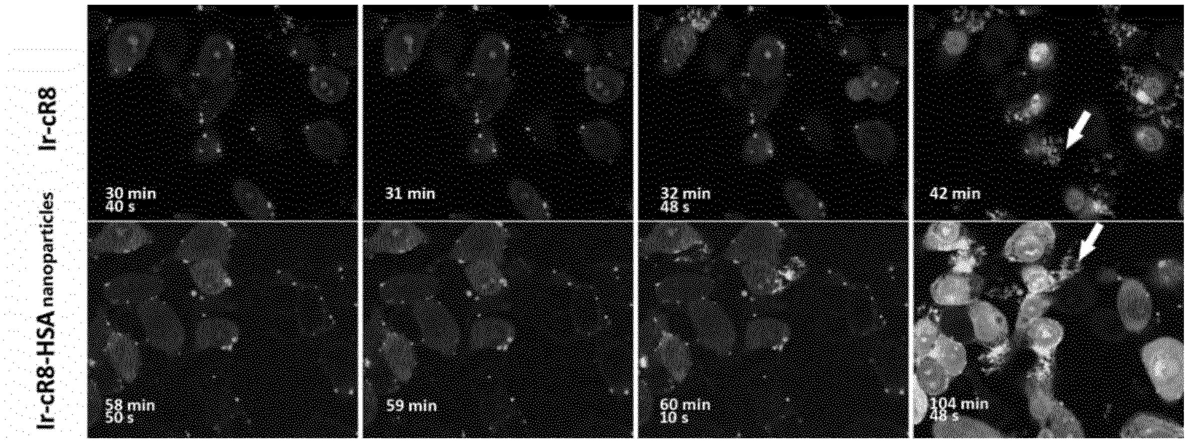


FIG. 20

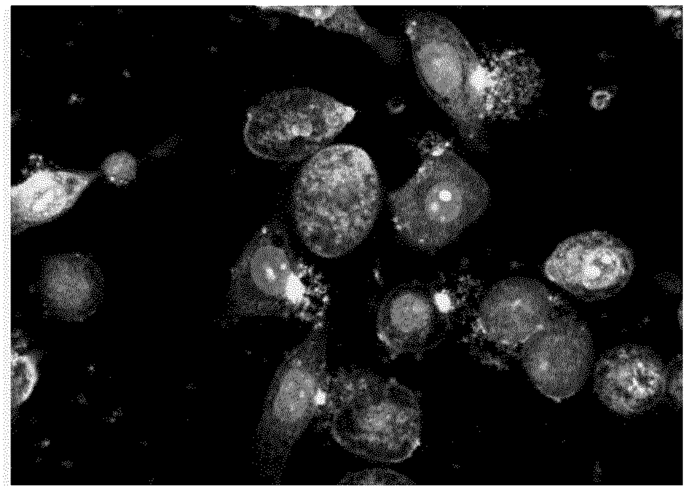

FIG. 21

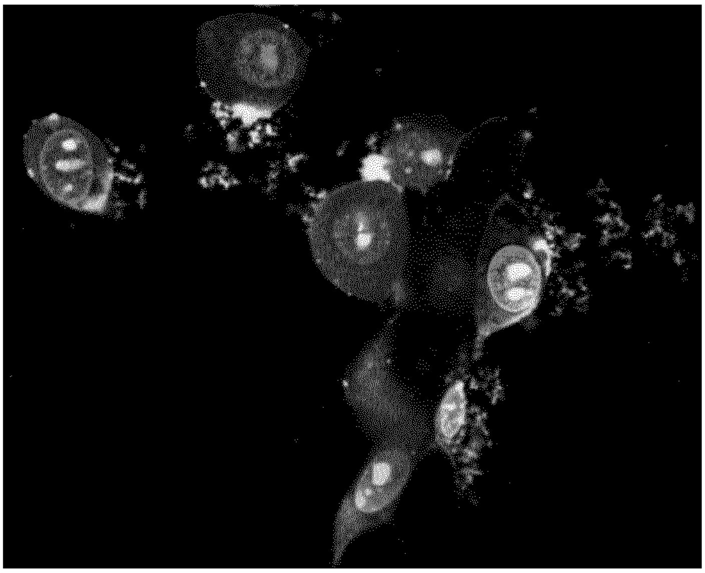

FIG. 22

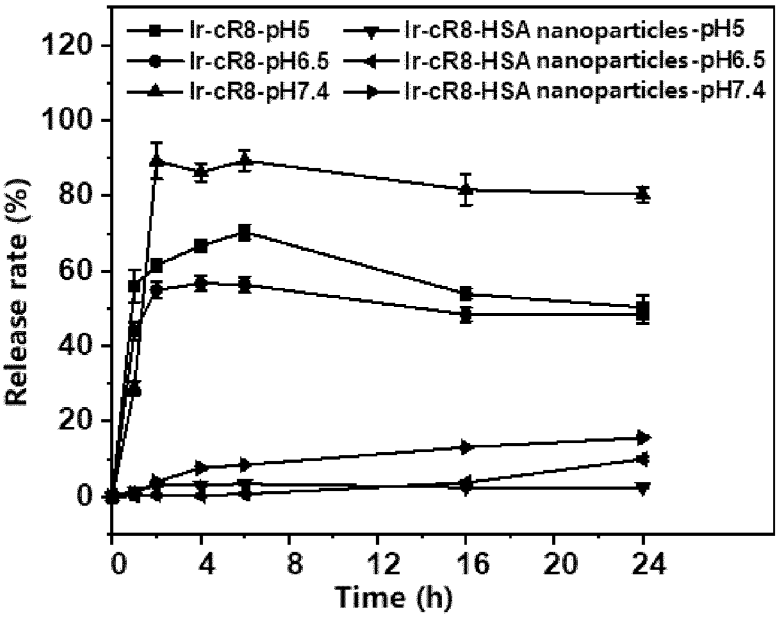
Ir-cR8-pH5
Ir-cR8-pH6.5
Ir-cR8-pH7.4
Ir-cR8-HSA nanoparticles-pH5
Ir-cR8-HSA nanoparticles-pH6.5
Ir-cR8-HSA nanoparticles-pH7.4
Release rate (%)
Time (h)
120
100
80
60
40
20
0
0
4
8
12
16
20
24

FIG. 23

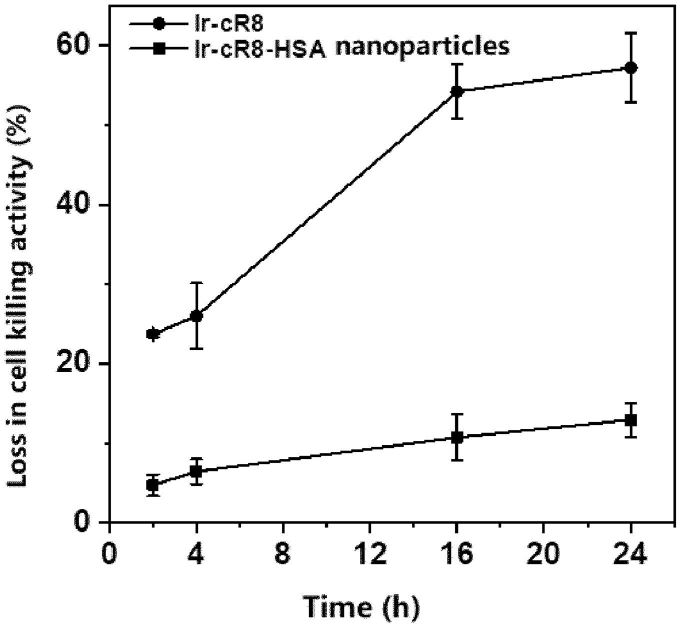
Ir-cR8
Ir-cR8-HSA nanoparticles
Loss in cell killing activity (%)
60
40
20
0
0
4
8
12
16
20
24
Time (h)

FIG. 24

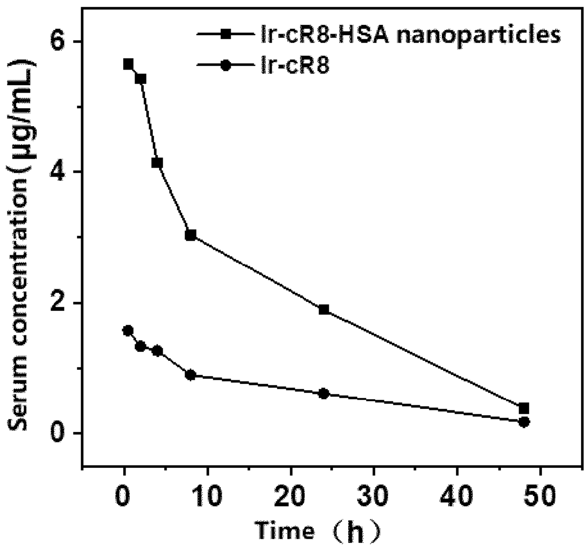
Ir-cR8-HSA nanoparticles
Ir-cR8
Serum concentration (μg/mL)
6
4
2
0
0
10
20
30
40
50
Time (h)

FIG. 25

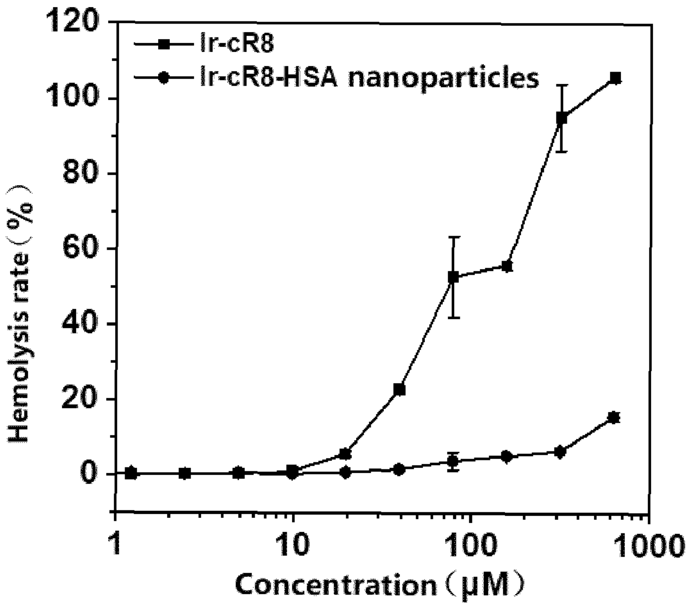
Ir-cR8
Ir-cR8-HSA nanoparticles
Hemolysis rate（%）
Concentration（μM）
120
100
80
60
40
20
0
1
10
100
1000

FIG. 26

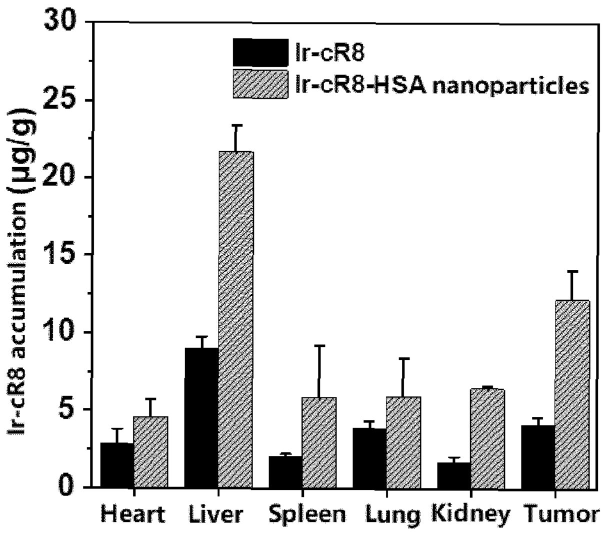
Ir-cR8
Ir-cR8-HSA nanoparticles
Ir-cR8 accumulation (μg/g)
30
25
20
15
10
5
0
Heart
Liver
Spleen
Lung
Kidney
Tumor

FIG. 27

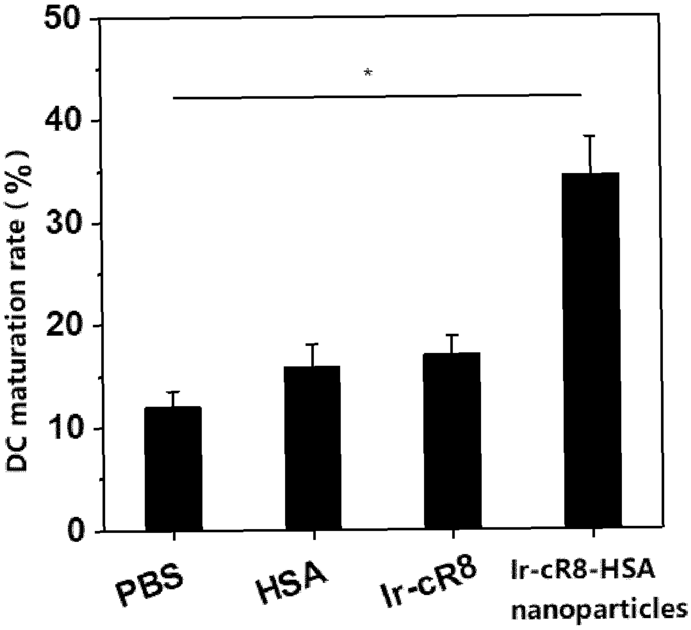
DC maturation rate (%)
50
40
30
20
10
0
*
PBS
HSA
Ir-cR8
Ir-cR8-HSA nanoparticles

FIG. 28

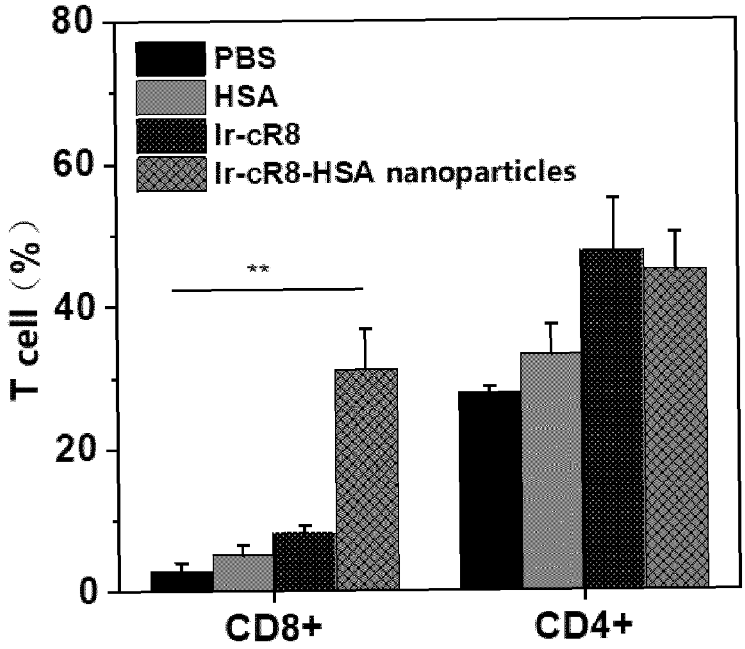
T cell (%)
80
60
40
20
0
PBS
HSA
Ir-cR8
Ir-cR8-HSA nanoparticles
**
CD8+
CD4+

FIG. 29

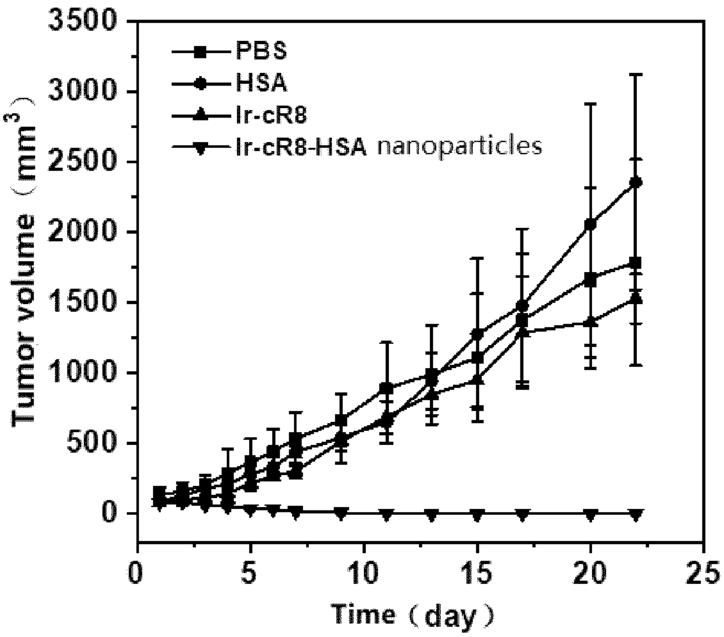
Tumor volume（mm3）
Time（day）
PBS
HSA
Ir-cR8
Ir-cR8-HSA nanoparticles
3500
3000
2500
2000
1500
1000
500
0
0
5
10
15
20
25

FIG. 30

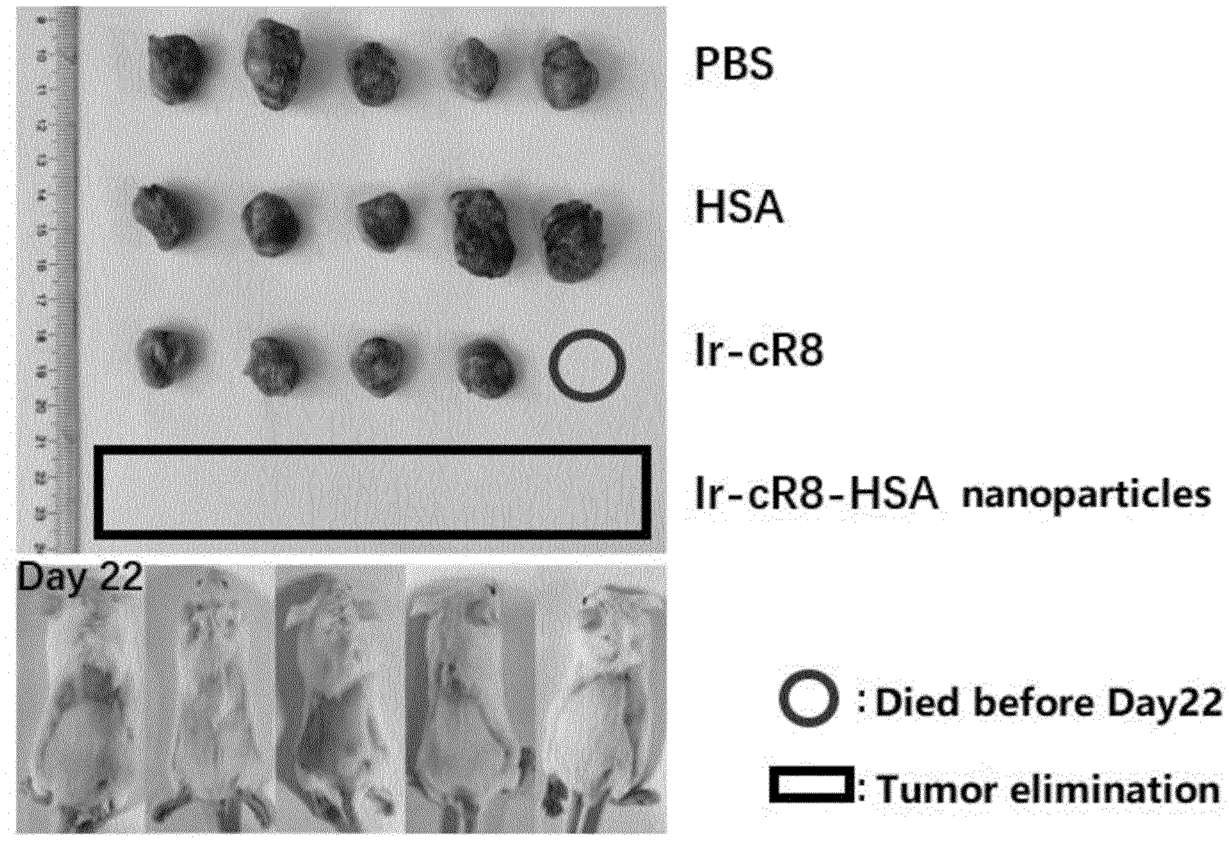
PBS
HSA
Ir-cR8
Ir-cR8-HSA nanoparticles
Day 22
: Died before Day22
: Tumor elimination

FIG. 31

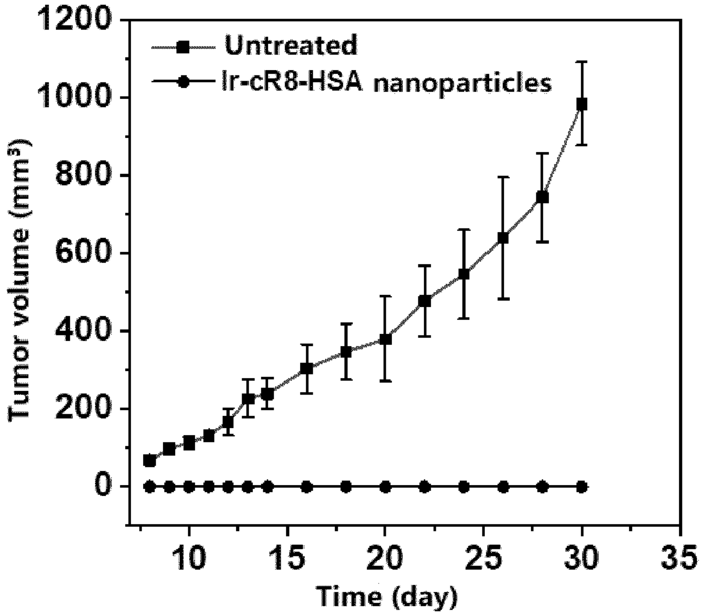
Untreated
Ir-cR8-HSA nanoparticles
Tumor volume (mm³)
1200
1000
800
600
400
200
0
10
15
20
25
30
35
Time (day)

FIG. 32

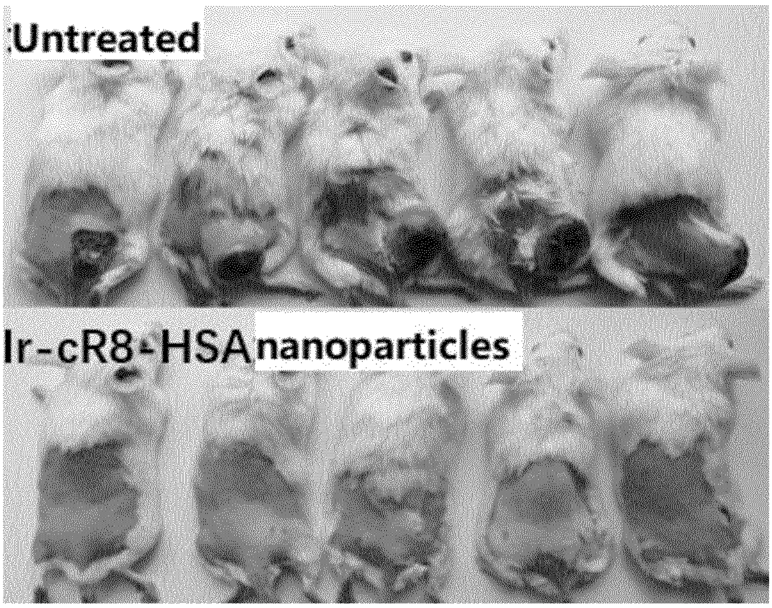
Untreated
Ir-cR8-HSA nanoparticles

FIG. 33

**INTERNATIONAL SEARCH REPORT**

International application No.
**PCT/CN2021/128313**

**A. CLASSIFICATION OF SUBJECT MATTER**

A61K 47/42(2017.01)i；A61K 9/51(2006.01)i；A61P 35/00(2006.01)i；B82Y 40/00(2011.01)i

According to International Patent Classification (IPC) or to both national classification and IPC

**B. FIELDS SEARCHED**

Minimum documentation searched (classification system followed by classification symbols)

A61K A61P B82Y

Documentation searched other than minimum documentation to the extent that such documents are included in the fields searched

Electronic data base consulted during the international search (name of data base and, where practicable, search terms used)

CNABS; CNTXT; SIPOABS; DWPI; USTXT; WOTXT; EPTXT; CNKI; ISI Web of Science; STNext: 费浩, 计双双, 肽, 抗菌肽, 阳离子, 正电荷, 正电, 双亲, 两亲, 疏水, 亲水, 白蛋白, 血清蛋白, 纳米粒, Peptide, cation, positive charge, amphiphilic, hydrophobic, hydrophilic, albumin, serum protein, nanoparticles, Transmembrane peptide, LL-37, TAT, CPPs, BSA, HSA

**C. DOCUMENTS CONSIDERED TO BE RELEVANT**

| Category* | Citation of document, with indication, where appropriate, of the relevant passages | Relevant to claim No. |
|---|---|---|
| PX | 计双双 (JI, Shuangshuang). "膜活性铱复合寡聚精氨酸多肽的抗肿瘤研究 (Anti-tumor Study of Membrane Active Iridium-coordinated Oligoarginine Peptides)" *《中国优秀博硕士学位论文全文数据库(博士) 医药卫生科技辑》 (Chinese Doctoral Dissertations Full-text Database, Medicine & Public Health),* No. 09, 15 September 2021 (2021-09-15), E079-2 abstract | 1-13 |
| X | CN 110496229 A (SHANGHAI PULMONARY HOSPITAL) 26 November 2019 (2019-11-26) description, paragraphs [0009]-[0015] | 1-13 |
| A | 丁静 等 (DING, Jing et al.). "人源抗菌肽LL-37在创伤修复中的研究进展 (Non-official translation: Advances in Human Cathelicidin LL-37 in Wound Healing)" *广东医学 (Guangdong Medical Journal),* Vol. 32, No. 18, ISSN: , pp. 2472-2475 | 1-13 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 December 2021** | **21 December 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/128313** |

**C. DOCUMENTS CONSIDERED TO BE RELEVANT**

| Category* | Citation of document, with indication, where appropriate, of the relevant passages | Relevant to claim No. |
|---|---|---|
| A | 季秀峰 等 (JI, Xiufeng et al.). "白蛋白纳米粒药物传递系统的研究进展 (Recent Advances of Albumin Nanoparticles as A Promising Drug Delivery System)" *沈阳药科大学学报 (Journal of Shenyang Pharmaceutical University),* Vol. 27, No. 12, ISSN: , pp. 968-978 | 1-13 |
| A | 刘安平 等 (LIU, Anping et al.). "抗菌肽LL-37的生物学活性及其作用 (Biological Activity and Effect of Antibacterial Peptide LL-37)" *医学信息 (Medical Information),* Vol. 32, No. 24, ISSN: , pp. 13-15 | 1-13 |

**INTERNATIONAL SEARCH REPORT**
**Information on patent family members**

International application No.
**PCT/CN2021/128313**

| Patent document cited in search report | Publication date (day/month/year) | Patent family member(s) | Publication date (day/month/year) |
|---|---|---|---|
| CN 110496229 A | 26 November 2019 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRAUN K ; POCHERT A ; LINDÉN MIKA et al.** Membrane interactions of mesoporous silica nanoparticles as carriers of antimicrobial peptides [J. *Journal of Colloid & Interface,* 2016, vol. 475, 161-170
- **HUANG C ; JIN H ; QIAN Y et al.** Hybrid melittin cytolytic Peptide-driven ultrasmall lipid nanoparticles block melanoma growth in vivo. [J. *ACS Nano,* 2013, vol. 7 (7), 5791-800
- **JI-GANG PIAO ; DONG LIU ; KAN HU et al.** Cooperative Nanoparticle System for Photothermal Tumor Treatment without Skin Damage [J]. *ACS Applied Materials & Interfaces,* 2016, vol. 8 (4), 5b11664